# DEMANDE DE BREVET EUROPEEN

(11) **EP 2 343 383 A1**
(43) Date de publication de la demande: **13.07.2011**
(21) Numéro de dépôt: 10170662.0
(22) Date de dépôt: 23.04.2007
(51) Int. Cl.: C12Q 1/68

(54) **Nouvelle sonde de détection agissant par reconnaissance moléculaire**

(30) Priorité: 25.04.2006 FR 0651440
(62) Demande divisionnaire de: 07731933.3
(71) Demandeur: Biomérieux, 69280 Marcy L'etoile (FR)
(72) Inventeur: Bernal-Mendez, Eloy, 38070, Saint Quentin Fallavier (FR); Laayoun, Ali, 38260 La Frette (FR); Laurent, Alain, 38100 Grenoble (FR)

(57) **Abrégé**

La présente invention concerne l'utilisation d'au moins une sonde de détection agissant par reconnaissance moléculaire d'une séquence cible, comprenant successivement dans le sens 5'-3',
■ un premier segment nucléotidique comprenant une structure stériquement encombrante à son extrémité 5', et
■ un deuxième segment nucléotidique, complémentaire de la séquence cible, pour bloquer une activité 5' nucléase d'une enzyme polymérase thermostable lors d'une Réaction de Polymérisation en Chaîne, la structure stériquement encombrante étant une structure en double brin.

## Description

La présente invention concerne la détection des acides nucléiques, notamment en Réaction de Polymérisation en Chaîne (PCR).

Il est souvent nécessaire, dans les technologies relatives aux acides nucléiques et au matériel génétique, de déterminer si un gène, une partie de gène ou une séquence nucléotidique est présent dans un organisme vivant, un extrait cellulaire de cet organisme ou tout autre échantillon biologique, alimentaire, industriel. L'intérêt de la recherche de séquences nucléotidiques spécifiques est immense, notamment pour la détection d'organismes pathogènes, la détermination d'anomalies génétiques par la présence d'allèles, la détection de la présence de lésions dans un génome hôte et la détection de l'expression génique par la présence d'un ARNm particulier ou de la modification d'un hôte cellulaire. De nombreuses maladies génétiques peuvent ainsi être diagnostiquées par l'analyse, et même la quantification, de l'expression de certains gènes.

Différents types de méthodes de détection spécifique des acides nucléiques sont décrits dans la littérature. Ces méthodes reposent généralement sur les propriétés d'appariement des brins complémentaires d'acides nucléiques, communément appelée « hybridation d'acides nucléiques » ou simplement « hybridation ».

D'une manière générale, après avoir déterminé la séquence spécifique d'un organisme ou d'une maladie qui doit être analysée, il convient d'extraire les acides nucléiques d'un échantillon, et éventuellement d'amplifier et de détecter la séquence d'intérêt. De nombreuses méthodes d'amplification et de détection ont été développées dans ce but. Ainsi, la PCR (Réaction de Polymérisation en Chaîne ou Polymerase Chain Reaction) est basée sur la répétition d'un processus en trois étapes : la dénaturation de l'ADN double brin pour obtenir deux simples brins complémentaires séparés, l'hybridation de chaque amorce à un brin d'ADN simple brin, et l'extension enzymatique des amorces par une ADN polymérase thermostable, par exemple la Taq DNA polymérase qui synthétise un brin d'ADN, ou amplicon, de séquence complémentaire à celui servant de cible. L'amplification peut être analysée en fin de cycles (PCR « end point »), ou suivie en temps réel (PCR en temps réel) grâce à la détection par fluorescence des amplicons : l'amplification et la détection des amplicons peuvent être donc séquentielle ou simultanées. Il existe plusieurs techniques de PCR en temps réel. L'une d'elles met en oeuvre des sondes particulières en épingle à cheveux appelées « moleculars beacons », comprenant une partie « boucle », complémentaire de l'amplicon cible à analyser, et deux parties « tige », complémentaires l'une de l'autre . Les « moleculars beacons » sont des séquences nucléotidiques comprenant un fluorophore à une extrémité d'une des parties « tige » et un extincteur de fluorescence (ou « quencher ») à l'extrémité de l'autre partie « tige ». En l'absence d'amplicon cible complémentaire, ces « molecular beacons » adoptent une configuration dite en « épingle à cheveux »: les extrémités 5' et 3' sont proches de part l'hybridation des deux parties « tige » : il n'y a pas d'émission de fluorescence. En présence d'amplicon, la partie « boucle » s'hybride à l'amplicon, ce qui provoque l'éloignement des deux parties « tige », le « quencher» ne joue plus son rôle et il y a émission de fluorescence.

Toutefois, lors d'une PCR, l'éloignement du fluorophore et du « quencher », qui permet l'émission d'un signal détectable, peut être dû non pas à l'hybridation de la sonde sur l'amplicon, mais à une dégradation de la sonde par l'enzyme polymérase thermostable, utilisée lors de la réaction d'amplification. En effet, les enzymes polymérase thermostables, et notamment l'enzyme Taq polymérase, ont une activité 5' nucléase. De ce fait, le signal émis à chaque cycle n'est plus directement proportionnel à la quantité d'amplicons produits (signal généré par la reconnaissance moléculaire entre le « molecular beacon » et l'amplicon), mais sera aussi fonction du clivage dû à l'enzyme polymérase thermostable. Le signal n'est donc plus quantitatif. De plus, outre l'émission de fluorescence « parasite » qui apparaît lors d'une PCR en temps réel, le clivage des « moleculars beacons » du à la présence résiduelle d'enzyme thermostable polymérase en fin de cycle affecte une analyse de gradient de température postérieure à l'amplification. Or cette analyse de gradient de température postérieure à l'amplification est la méthode la plus sensible pour détecter des variations simples des séquences, comme les SNP (« single nucleotide polymorphism », ou polymorphisme d'un seul nucléotide) par les « molecular beacons ».

Il est donc très important d'augmenter la sensibilité de la technique PCR, en empêchant tout clivage parasite par les enzymes thermostables polymérases utilisées lors d'une PCR, en particulier l'enzyme Taq Polymérase.

La présente invention se propose de résoudre l'ensemble des inconvénients de l'état de la technique en améliorant la sensibilité et la fiabilité de la détection en PCR, en temps réel ou « end-point ». A ce titre, l'invention concerne de nouveaux oligonucléotides marqués résistants à l'activité 5' nucléase des enzymes polymérases thermostables telles que la Taq polymérase.

A cet effet, la présente invention concerne une sonde de détection agissant par reconnaissance moléculaire d'une séquence cible, comprenant successivement dans le sens 5'-3',
■ un premier segment nucléotidique comprenant une structure stériquement encombrante à son extrémité 5' et
■ un deuxième segment nucléotidique, complémentaire de la séquence cible. Selon un mode préféré de réalisation de l'invention, la structure stériquement encombrante est une structure en arborescence, en épingle à cheveux ou en double brin.

Selon un mode préféré de réalisation de l'invention, la sonde de détection agissant par reconnaissance moléculaire comprend en outre, en aval dudit deuxième segment nucléotidique, un troisième segment nucléotidique, capable de s'hybrider sur ledit premier segment nucléotidique.

Selon un mode préféré de réalisation de l'invention, la sonde de détection agissant par reconnaissance moléculaire est marquée, à une extrémité par un fluorophore et à l'autre extrémité, par un extincteur.

Préférentiellement, ledit troisième segment est complémentaire en tout ou partie dudit premier segment de façon à ce que, sous de conditions de température et salinité favorables, notamment les conditions des étapes d'hybridation et de polymérisation dans une réaction de PCR, ces deux parties complémentaires interagissent entre elles, formant une double hélice intra-moléculaire.

Selon un mode préféré de réalisation de l'invention, ledit premier segment comprend un fluorophore et ledit troisième segment comprend un extincteur. Selon un autre mode préféré de réalisation de l'invention, ledit premier segment comprend un extincteur et ledit troisième segment comprend un fluorophore.

Selon un mode préféré de réalisation de l'invention, lesdits premier et/ou troisième segments comprennent préférentiellement de 3 à 8 nucléosides, encore plus préférentiellement de 4 à 6 nucléosides.

Selon un mode préféré de réalisation de l'invention, ledit deuxième segment comprend préférentiellement de 10 à 35 nucléosides et encore plus préférentiellement de 15 à 25 nucléosides

Les définitions suivantes permettront de mieux comprendre l'invention.

Au sens de la présente invention, on entend par « amont », une région située du coté de l'extrémité 5' de l'acide nucléique ou de la séquence polynucléotidique, et par « aval » une région située du coté de l'extrémité 3' dudit acide nucléique ou de ladite région polynucléotidique.

Au sens de la présente invention, les termes fragment nucléotidique, fragments d'acide nucléique, segment nucléotidique, segment d'acide nucléique, séquence nucléotidique, séquence d'acide nucléique, ou oligonucléotide, désignent un fragment d'ADN ou d'ARN naturel, un polynucléotide naturel ou de synthèse, un fragment d'ADN ou d'ARN de synthèse non modifiée ou comprenant au moins une modification, que ce soit sur la nucléobase (base modifiée telles que l'inosine, la méthyl-5-désoxycitidine, la diméthylamino-5-desoxyuridine, la désoxyuridine, la diamino 2,6 purine, la bromo-5 désoxyuridine, la pseudouridine, la pseudoisocytidine, ou toute autre base modifiée), sur le sucre (comme les 2'-OMe, Locked Nucleic Acids, nucléotides alpha ou autres) ou sur le phosphate (phosphorothioate, phosphoramidate et autres). Chacune des modifications peut être prise en combinaison.

Par sonde de détection agissant par reconnaissance moléculaire, on entend une séquence nucléique de 10 à 100 motifs nucléotidiques, notamment de 15 à 45 motifs nucléotidiques, possédant une spécificité d'hybridation dans des conditions déterminées pour former un complexe d'hybridation avec une séquence nucléique cible et émettant un signal lors de l'hybridation de la sonde sur la séquence nucléique cible.

La sonde de détection peut être notamment une sonde de détection « molecular beacon » telle que décrite par Tyagi & Kramer (Nature Biotech, 1996, 14 :303-308). Ces « molecular beacons » deviennent fluorescentes lors de l'hybridation. Elles possèdent une structure de type tige-boucle et contiennent un fluorophore et un groupe « quencher ». La fixation de la séquence de boucle spécifique avec sa séquence complémentaire d'acide nucléique cible provoque un déroulement de la tige et l'émission d'un signal fluorescent lors de l'excitation à la longueur d'onde qui convient.

La sonde de détection peut être notamment une sonde de détection « Scorpion® » ou « Amplifluor® » Les Scorpions® sont des séquences qui possèdent du côté 5' une séquence avec une structure tige-boucle avec un "quencher" à une extrémité de la dite structure et un fluorophore à l'autre extrémité de la dite structure. Du côté 3', les Scorpions® possèdent une séquence qui est complémentaire d'une séquence de la cible et qui agit en tant qu'amorce lors de la réaction d'amplification. Entre la séquence amorce et la séquence tige-boucle, un « espaceur » permet d'empêcher la reconnaissance de la séquence tige-boucle par la polymérase. Un « espaceur » est une molécule synthétique qui est incorporé dans une séquence nucléotidique pendant la synthèse pour séparer ladite séquence en deux parties, afin de produire des effets différents selon sa nature.

Les Amplifluor® sont des séquences nucléotidiques qui possèdent du côté 5' une séquence avec une structure tige-boucle, avec un fluorophore à l'extrémité 5', et un "quencher" de type Dabsyl à l'autre extrémité de la structure. Du côté 3', les Amplifuor® possèdent une séquence qui est complémentaire d'une séquence de la cible et qui agit en tant qu'amorce lors de la réaction d'amplification.

Au sens de la présente invention, on entend par « fluorophore » une molécule qui émet un signal de fluorescence entre 500 et 700nm quand elle est excitée par de la lumière à une longueur d'onde qui convient (ou entre 450 et 650 nm). Le fluorophore peut être notamment une rhodamine ou un dérivé tel que Texas Red, une fluorescéine ou un dérivé, tel que la 5 bromométhyl-fluorescéine, un fluorophore de la famille des Alexa tels que l'Alexa532 et l'Alexa647, Alexa 405, Alexa 700, Alexa 680 , ou tout autre fluorophore qui convienne en fonction de l'appareil de mesure utilisé. Les fluorophores disponibles pour les sondes de détection sont très variés et connus de l'homme du métier.

Au sens de la présente invention, on entend par « fluorescéine » une molécule chimique aromatique qui émet un signal de fluorescence avec un maximum d'émission autour de 530 nm, quand elle est excitée par de la lumière à une longueur d'onde autour de 495 nm.

Au sens de la présente invention, on entend par « extincteur » (ou ""quencher"") une molécule qui interfère avec la fluorescence émise par un fluorophore. Cet extincteur est notamment choisi parmi des molécules aromatiques non fluorescentes, pour éviter des émissions parasites. Préférentiellement, le dit « extincteur » est un Dabsyl ou un Dabcyl ou un « Black hole quencher™ » . Le Dabcyl, le Dabsyl et les « Black hole quencher™ » sont des molécules aromatiques non fluorescentes qui empêchent l'émission de fluorescence quand elles se trouvent physiquement à proximité d'un fluorophore, ou par FRET.

Au sens de la présente invention, on entend par « segment nucléotidique, capable de s'hybrider sur une séquence cible», une séquence ou une région pouvant s'hybrider sur une autre séquence/région dans des conditions d'hybridation, qui peuvent être déterminées dans chaque cas de façon connue. On parle également de séquences/régions complémentaires. Une séquence ou région strictement complémentaire d'une autre est une séquence dans laquelle chacune des bases peut s'apparier avec une base de l'autre séquence, sans mésappariement.

Par hybridation, on entend le processus au cours duquel, dans des conditions appropriées, deux fragments nucléotidiques, ayant des séquences suffisamment complémentaires sont susceptibles de former un double brin avec des liaisons hydrogènes stables et spécifiques. Les conditions d'hybridation sont déterminées par la stringence, c'est-à-dire la rigueur et la faible salinité des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier.

Au sens de la présente invention, on entend par « structure stériquement encombrante » une structure, notamment nucléotidique, qui impose une contrainte stérique capable de bloquer l'activité 5'-nucléase d'une enzyme polymérase thermostable tel qu'une enzyme DNA Polymérase, responsable de la dégradation d'un oligonucléotide, c'est à dire une structure ayant une taille supérieure à celle d'un simple brin d'ADN. Préférentiellement, la structure stériquement encombrante a au moins la taille d'une double hélice d'ADN, c'est à dire un diamètre minimum d'au moins 20 angströms.

Selon un mode préféré de réalisation de l'invention, la structure stériquement encombrante est une structure en épingle à cheveux. L'avantage d'une telle structure est qu'elle peut être faite sans avoir recours à des réactifs autres que ceux utilisés pour la synthèse normale des sondes nucléotidiques ADN et sans modification des cycles de synthèse. Cette structure peut ainsi être obtenue par synthèse linéaire. Elle est pratique du point de vue de la caractérisation et contrôle qualité car on peut utiliser les mêmes techniques analytiques que pour les oligonucleotides et sondes classiques. De plus, du fait de sa facilité de synthèse et de sa simplicité, il est aisé pour l'homme du métier de moduler cette structure selon les besoins en termes de longueur et de séquence nucléotidique. La longueur de séquence nécessaire à la formation d'une telle structure est celle qui lui permet de rester stable dans les conditions de salinité et à la température de travail de la DNA Polymérase pendant la réaction de polymérisation dans la PCR. La taille de la structure, dans le sens de l'encombrement, est celle d'une double hélice.

Par structure en épingle à cheveux, on entend une séquence nucléotidique linéaire, naturelle ou modifiée dans laquelle une partie de la séquence est complémentaire d'une autre partie de la séquence, de façon à ce que, sous de conditions de température et salinité favorables, notamment les conditions des étapes d'hybridation et de polymérisation dans une réaction de PCR, ces deux parties complémentaires interagissent entre elles formant une double hélice intra-moléculaire.

Selon un autre mode préféré de réalisation de l'invention, la structure stériquement encombrante est une structure en double brin. L'avantage d'une telle structure est qu'elle peut être faite sans avoir recours à des réactifs autres que ceux utilisés pour la synthèse normale des sondes nucléotidiques ADN et sans modification des cycles de synthèse. Par structure en double brin, on entend deux séquences nucléotidiques complémentaires qui s'hybrident entre elles pour former une double hélice sous de conditions de température et salinité favorables, notamment les conditions des étapes d'hybridation et de polymérisation dans une réaction de PCR. Par rapport à la structure en épingle à cheveux, une structure en double brin permet d'avoir moins de nucléotides à ajouter en 5' de la sonde nucléique, ce qui fait améliore le rendement de synthèse et de purification. Le deuxième brin qui forme le double brin est une autre molécule qui peut-être synthétisée séparément.

L'invention concerne également l'utilisation d'une structure stériquement encombrante pour bloquer une activité 5' nucléase d'une enzyme polymérase.

L'invention concerne également l'utilisation d'au moins une sonde de détection agissant par reconnaissance moléculaire tel que définie précédemment et qui bloque l'activité 5' nucléase d'une enzyme polymérase thermostable lors d'une Réaction de Polymérisation en Chaîne.

De telles utilisations sont très pertinentes puisqu'on peut ainsi détecter une séquence cible par l'utilisation de sondes de type « molecular beacon » tout en évitant le clivage de celles-ci par l'activité 5' nucléase d'une enzyme polymérase lors d'une Réaction de Polymérisation en Chaîne, en temps réel ou lors de mesures en « end-point » post-amplification.

L'invention concerne également un procédé pour détecter un matériel nucléique dans un échantillon biologique comprenant les étapes suivantes :
a) on extrait le matériel nucléique d'un échantillon biologique,
b) on amplifie le matériel nucléique pour obtenir des amplicons d'au moins une séquence cible du matériel nucléique en présence d'une enzyme polymérase
c) on utilise, simultanément à l'étape b) d'amplification ou postérieurement à l'étape b) d'amplification, au moins une sonde de détection agissant par reconnaissance moléculaire tel que décrit précédemment
d) on détecte la présence desdits amplicons

Au sens de la présente invention, on entend par « matériel nucléique », une séquence d'acides nucléiques telle que une séquence d'acides désoxyribonucléiques (ADN) ou d'acides ribonucléiques (ARN). Selon un mode préféré de réalisation de l'invention, le matériel nucléique comprend une séquence d'acides désoxyribonucléiques. Selon un mode préféré de réalisation de l'invention, le matériel nucléique est extrait d'un échantillon biologique prélevé chez un patient.

Au sens de la présente invention, on entend par « échantillon biologique », tout échantillon susceptible de contenir un matériel nucléique tel que défini ci après. Cet échantillon biologique peut être prélevé chez un patient et peut être notamment un échantillon de tissus, de sang, de sérum, de salive, de cellules circulantes du patient. Cet échantillon peut également être un échantillon alimentaire. On dispose de cet échantillon biologique par tout type de prélèvement connu de l'homme du métier. Au sens de la présente invention, on entend par « séquence cible », une séquence nucléotidique dont au moins une partie de l'enchaînement en motifs nucléotidiques est spécifique et complémentaire de la séquence nucléotidique de la sonde de détection utilisée.

Au sens de la présente invention, on entend par « enzyme polymérase thermostable » une enzyme naturelle ou modifiée qui est capable de synthétiser un brin d'ADN de séquence complémentaire de celle présente dans le milieu réactionnel, qui est utilisée comme cible ou modèle, à partir du côté 3' d'une amorce de séquence complémentaire à la cible, et utilisant des nucléotides triphosphate comme substrat et capable de supporter, sans perdre son activité biologique, de hautes températures, notamment jusqu'a 95°C pendant quelques minutes.

Au sens de la présente invention, lors de l'étape a) on extrait le matériel nucléique d'un échantillon biologique par tout protocole connu de l'homme du métier. A titre indicatif, l'extraction d'acides nucléiques peut être réalisée par une étape de lyse des cellules présentes dans l'échantillon biologique, afin de libérer les acides nucléiques contenus dans les enveloppes protéiques et/ou lipidiques des cellules (comme des débris cellulaires qui perturbent les réactions ultérieures). A titre d'exemple, on peut utiliser les méthodes de lyse telles que décrite dans les demandes de brevet WO 00/05338 sur la lyse mixte magnétique et mécanique, WO 99/53304 sur la lyse électrique, et WO 99/15321 sur la lyse mécanique.

L'homme du métier pourra utiliser d'autres méthodes de lyse bien connues, telles que les chocs thermiques ou osmotiques ou les lyses chimiques par des agents chaotropiques tels que les sels de guanidium (US 5,234,809). Cette étape de lyse peut également être suivie d'une étape de purification, permettant la séparation entre les acides nucléiques et les autres constituants cellulaires relargués dans l'étape de lyse. Cette étape permet généralement de concentrer les acides nucléiques, et peut être adapté à la purification d'ADN ou d'ARN. A titre d'exemple, on peut utiliser des particules magnétiques éventuellement revêtues d'oligonucléotides, par adsorption ou covalence (voir à ce sujet les brevets US 4,672,040 et US 5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet WO 97/45202 et WO 99/35500. Un autre exemple intéressant de méthode de purification des acides nucléiques est l'utilisation de silice soit sous forme de colonne, soit sous forme de particules inertes (Boom R. et al., J. Clin. Microbiol., 1990, n°28(3), p. 495-503) ou magnétiques (Merck: MagPrep® Silica, Promega: MagneSil^{™} Paramagnetic particles). D'autres méthodes très répandues reposent sur des résines échangeuses d'ions en colonne ou en format particulaire paramagnétique (Whatman: DEAE-Agarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-Bind^{™}).

Lorsque l'on souhaite extraire spécifiquement l'ADN d'un échantillon biologique, on peut notamment réaliser une extraction par du phénol, du chloroforme et de l'alcool pour éliminer les protéines et précipiter l'ADN avec de l'alcool 70%. L'ADN peut alors être culoté par centrifugation, lavé et remis en suspension.

Au sens de la présente invention, 1'« étape b) » est un processus générant de multiples copies (ou amplicons) d'une séquence nucléique par l'action d'au moins une enzyme polymérase. Au sens de la présente invention, on entend par amplicons les copies de la séquence cible obtenues lors d'une réaction d'amplification enzymatique. De telles réactions d'amplification sont bien connues de l'homme du métier et on peut citer notamment la PCR (Polymerase Chain Reaction), telle que décrite dans les brevets US-A-4,683,195, US-A-4,683,202 et US-A-4,800,159.

D'une manière générale, l'étape b) est une succession de cycles comprenant les étapes suivantes :
o la dénaturation de la séquence cible afin d'obtenir deux brins cibles d'ADN complémentaires, ou de déstructurer le brin cible d'ARN,
o l'hybridation de chacun des brins cibles, obtenus lors de l'étape de dénaturation précédente avec au moins une amorce d' amplification ,
o la formation à partir des amorces d'amplification des brins complémentaires aux brins sur lesquels elles sont hybridées en présence d'une enzyme polymérase et de nucléosides triphosphate
ce cycle étant répété un nombre de fois déterminé pour obtenir la séquence cible dans une concentration suffisante pour permettre sa détection.

Les étapes b) et c) sont effectuées en même temps ou l'une après l'autre.

Lorsque les étapes b) et c) sont effectuées en même temps, ce mode de réalisation est préférentiellement mise en oeuvre par « PCR en temps réel » qui regroupe en une étape unique la technique d'amplification PCR et la détection, et qui fait appel notamment à des « molecular beacons ». La réaction PCR intervient dans le tube, produisant des amplicons avec lequel les « molecular beacons » spécifiques peuvent s'hybrider pour donner un signal fluorescent. La formation des nouvelles molécules d'ADN est mesurée en temps réel par contrôle du signal dans un lecteur fluorescent, pendant l'étape d'hybridation. L'utilisation d'oligonucléotides marqués selon la présente invention permet d'éviter que les sondes de détection soient dégradées par l'enzyme d'amplification (par exemple la Taq Polymérase), ce qui augmente la sensibilité de la détection et améliore les performances de la technique.

Lorsque les étapes b) et c) sont effectuées l'une après l'autre, la réaction PCR intervient dans le tube, produisant des amplicons. A la fin de cette étape d'amplification, les « molecular beacons » sont ajoutés au milieu de réaction, et peuvent s'hybrider pour donner un signal fluorescent. L'utilisation d'oligonucléotides marqués selon la présente invention permet d'éviter que les sondes de détection soient dégradées par l'enzyme polymérase thermostable d'amplification, qui reste de manière résiduelle dans le tube de réaction, ce qui augmente la sensibilité de la détection et améliore les performances de la technique.

L'« étape d) » est réalisée par la détection du signal de fluorescence émis lors de l'hybridation de l'oligonucléotide marqué selon l'invention sur l'amplicon, et peut être réalisée par tous protocoles connus de l'homme du métier.

Les figures ci-jointes sont données à titre d'exemple explicatif et n'ont aucun caractère limitatif. Elles permettront de mieux comprendre l'invention.

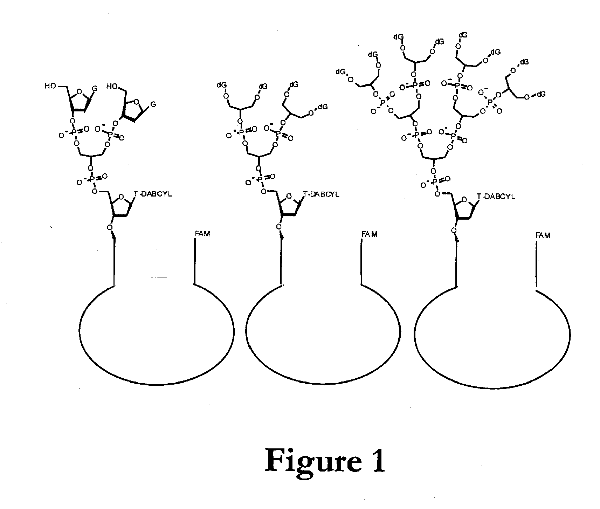

La figure 1 présente des « molecular beacons » selon l'invention, comprenant une structure stériquement encombrante en arborescence.

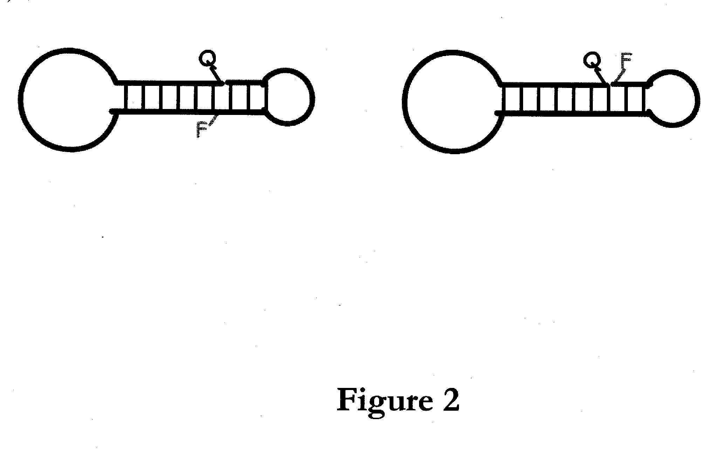

La figure 2 présente des « molecular beacons » selon l'invention, comprenant du côté 5' une structure stériquement encombrante en épingle à cheveux. La ligne continue représente la séquence nucléotidique. La lettre "Q" représente le « quencher » situé à l'extrémité 3'. La lettre "F" représente le fluorophore situé sur une base T à l'intérieur de la séquence, dans le premier segment de la sonde (dessin de gauche) et le fluorophore situé à l'extrémité 5' de la séquence, dans la structure en épingle à cheveux (dessin de droite). Les lignes verticales représentent l'hybridation, dans les segments qui forment des doubles hélices.

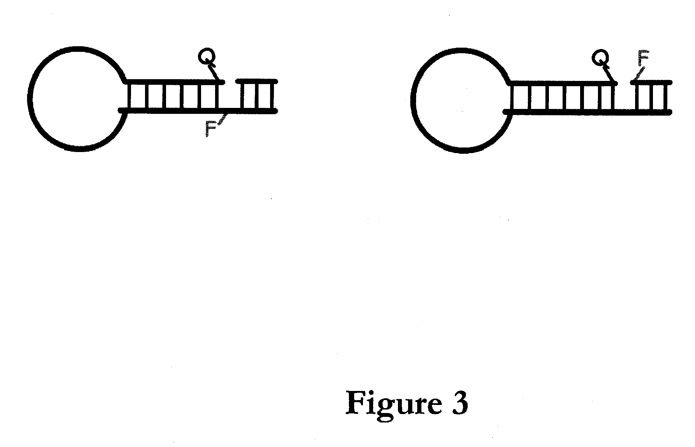

La figure 3 présente des « molecular beacons » selon l'invention comprenant du côté 5' une structure stériquement encombrante en double brin. Les lignes continues représentent les séquences nucléotidiques. La lettre "Q" représente le « quencher » situé à l'extrémité 3' de la séquence des « molecular beacons ». La lettre "F" représente le fluorophore situé sur une base T à l'intérieur de la séquence du « molecular beacon », dans le premier segment de la sonde (dessin de gauche) et le fluorophore situé à l'extrémité 5' de la séquence de l'oligonucléotide complémentaire qui sert à former la structure stériquement encombrante en double brin (dessin de droite). Les lignes verticales représentent l'hybridation, dans les segments qui forment des doubles hélices.
La figure 4 présente les profils de fluorescence mesurés à 40, 60 et 95°C, pour la sonde TaqMan tels que décrits dans l'exemple 1.
La figure 5 présente les profils de fluorescence mesurés à 40, 60 et 95°C, pour le contrôle négatif de la sonde TaqMan tels que décrits dans l'exemple 1.
La figure 6 présente les profils de fluorescence mesurés à 40, 60 et 95°C, pour le « molecular beacon » G8 tels que décrit dans l'exemple 1.
La figure 7 présente les profils de fluorescence mesurés à 40, 60 et 95°C, pour le contrôle négatif du « molecular beacon » G8 tels que décrit dans l'exemple 1.
La figure 8 présente les profils de fluorescence normalisés mesurés à 95°C pour le « molecular beacon » G8 et pour la sonde TaqMan TM pendant l'amplification sur Lightcycler tels que décrit dans l'exemple 1.
La figure 9 présente les profils de fluorescence mesurés à 40, 60 et 95°C, pour la sonde TaqMan, tel que décrit dans l'exemple 2.
La figure 10 présente les profils de fluorescence mesurés à 40, 60 et 95°C, pour le contrôle négatif de la sonde TaqMan, tel que décrit dans l'exemple 2.
La figure 11 présente les profils de fluorescence mesurés à 40, 60 et 95°C, pour le « molecular beacon » hp5, tel que décrit dans l'exemple 2.
La figure 12 présente les profils de fluorescence mesurés à 40, 60 et 95°C, pour le contrôle négatif du « molecular beacon » hp5, tel que décrit dans l'exemple 2.
La figure 13 présente les profils de fluorescence normalisés mesurés à 95°C pour le « molecular beacon » hp5 et la sonde TaqMan TM pendant l'amplification sur Lightcycler, tel que décrit dans l'exemple 2.
La figure 14 présente les profils de fluorescence mesurés à 40, 60 et 95°C, pour le « molecular beacon » contrôle tel que décrit dans l'exemple 3.
La figure 15 présente les profils de fluorescence mesurés à 40, 60 et 95°C, pour le contrôle négatif du « molecular beacon » contrôle, tel que décrit dans l'exemple 3.
La figure 16 présente les profils de fluorescence mesurés à 40, 60 et 95°C, pour le « molecular beacon » hp2, tel que décrit dans l'exemple 3.
La figure 17 présente les profils de fluorescence mesurés à 40, 60 et 95°C, pour le contrôle négatif du « molecular beacon » hp2, tel que décrit dans l'exemple 3.
La figure 18 présente les profils de fluorescence normalisés mesurés à 95°C pour le « molecular beacon » hp2 et le « molecular beacon » MBnm pendant l'amplification sur Lightcycler tels que décrit dans l'exemple 3.
La figure 19 présente les profils de fluorescence mesurés à 40, 60 et 95°C, pour la sonde TaqMan, tel que décrit dans l'exemple 4.
La figure 20 présente les profils de fluorescence mesurés à 40, 60 et 95°C, pour le contrôle négatif de la sonde TaqMan, tel que décrit dans l'exemple 4.
La figure 21 présente les profils de fluorescence mesurés à 40, 60 et 95°C, pour le « molecular beacon » hp4, tel que décrit dans l'exemple 4.
La figure 22 présente les profils de fluorescence mesurés à 40, 60 et 95°C, pour le contrôle négatif du « molecular beacon » hp4, tel que décrit dans l'exemple 4.
La figure 23 présente les profils de fluorescence normalisés mesurés à 95°C pour le « molecular beacon » hp4 et la sonde TaqMan TM pendant l'amplification sur Lightcycler, tel que décrit dans l'exemple 4.
La figure 24 présente les profils de fluorescence mesurés à 40, 60 et 95°C, pour la sonde TaqMan, tel que décrit dans l'exemple 5.
La figure 25 présente les profils de fluorescence mesurés à 40, 60 et 95°C, pour le contrôle négatif de la sonde TaqMan, tel que décrit dans l'exemple 5.
La figure 26 présente les profils de fluorescence mesurés à 40, 60 et 95°C, pour le « molecular beacon » hp3, tel que décrit dans l'exemple 5.
La figure 27 présente les profils de fluorescence mesurés à 40, 60 et 95°C, pour le contrôle négatif du « molecular beacon » hp3, tel que décrit dans l'exemple 5.

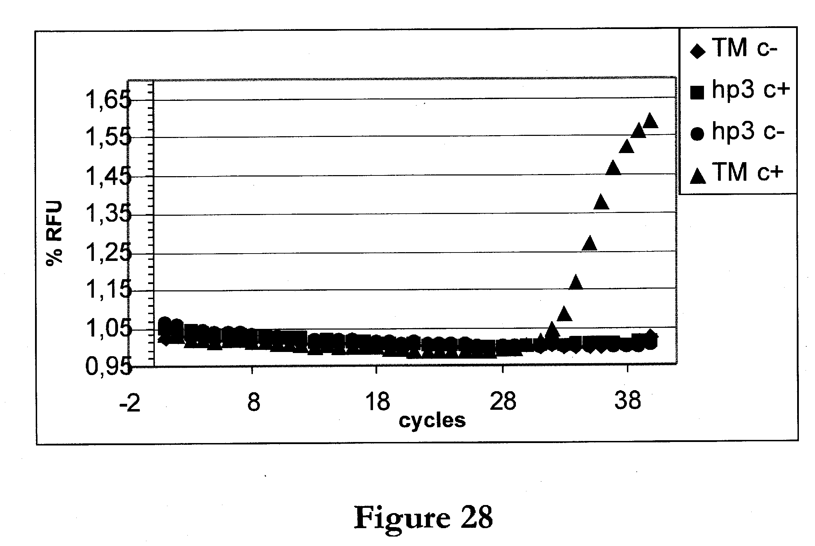

La figure 28 présente les profils de fluorescence normalisés mesurés à 95°C pour le « molecular beacon » hp3 et la sonde TaqMan TM pendant l'amplification sur Lightcycler, tel que décrit dans l'exemple 5.

Les exemples suivants sont donnés à titre illustratif et n'ont aucun caractère limitatif. Ils permettront de mieux comprendre l'invention.

### 1. Exemple de protection par une structure nucléotidique en arborescence contre le clivage par l'activité 5'-nucléase d'une sonde agissant par reconnaissance moléculaire.

L'objectif de cette expérience était de démontrer qu'une sonde modifié en 5' par une structure nucléotidique en arborescence n'est pas clivée par l'activité 5'-nucléase de la Taq Polymerase dans une réaction d'amplification enzymatique *in vitro.*

### 1.a : Définition de la cible, sondes et amorces

La cible était le plasmide pCITE avec insert de 1.2 Kb correspondant à une séquence génétique du virus hMPV. 5.10³ copies par tube.

Les séquences utilisées étaient les suivantes :
○ Amorce sens: 5'- CAT ATA AGC ATG CTA TAT TAA AAG AGT CTC -3'
○ Amorce reverse: 5'- CCT ATT TCT GCA GCA TAT TTG TAA TCA G -3'
○ Sonde "molecular beacon" (G8): 5'-G₈D₄-D₂-D-(dT-FAM)- CGA TGC AAC TGC AGT GAC ACC CTC ATC ATT GCA CAT CG (dT-Dabcyl)-3'
○ Sonde TaqMan (TM): 5'- FAM- TGC AAT GAT GAG GGT GTC ACT GCG GTT -TAMRA-3' - Cette séquence reconnaissait spécifiquement une séquence de l'amplicon généré dans l'amplification *in- vitro.*

La partie soulignée de cette séquence reconnaissait spécifiquement une séquence de l'amplicon généré dans l'amplification in vitro.

### 1.b : Synthèse de sondes et amorces

Les séquences de sondes bloquées en 5' et des amorces ont été synthétisées sur un appareil Expedite (Perseptive Biosystems) selon la méthode au phosphoramidite par voie automatique conformément au protocole proposé par le constructeur. Le réactif phosphoramidite nécessaires à l'introduction du motif « D » est un réactif commercial (réf. 10-1920, Glen Research, USA). Cette molécule possédait deux terminaisons réactivées, qui permettaient de coupler simultanément, dans le même cycle de synthèse, deux nucléotides (des G dans cet exemple) au lieu d'un nucléotide par cycle dans le cas des réactifs standards. En intégrant cette molécule trois fois dans la synthèse, une terminaison 5' qui possédait huit nucléotides en parallèle a été crée. Cette structure en arborescence était plus encombrante qu'une terminaison naturelle, et empêchait la polymérase de reconnaître l'extrémité 5' et de cliver le « molecular beacon ».

Le dT-FAM est un nucléotide modifié qui portait une molécule Fluorescéine attachée de façon covalente à la base T (ref. 10-1056, Glen Research, USA).

Le dT-Dabcyl était un nucléotide modifié qui portait une molécule Dabcyl attachée de façon covalente à la base T (ref. 10-1058, Glen Research, USA).

Les sondes et primers sont purifiées par HPLC en phase inverse, leur pureté est contrôlée par électrophorèse capillaire.

La molécule FAM est un fluorophore de type fluorescéine dont la fluorescence est détectée à 530 nm. La molécule Dabcyl est une molécule aromatique qui empêche l'émission de fluorescence quand elle se trouve physiquement à proximité du fluorophore FAM.

La molécule TAMRA (dérivé de la tétraméthylrhodamine) est une molécule aromatique qui empêche l'émission de fluorescence de la fluorescéine par effet FRET lorsque ces deux molécules sont situées à proximité, par exemple sur une même séquence nucléotidique. L'effet FRET est proportionnel à la sixième puissance de la distance entre les deux molécules.

### 1.c : Amplification PCR avec détection en temps réel sur LightCycler:

Dans cet exemple, un kit d'amplification « LightCycler FastStart DNA Master Hybridization Probes » (Roche, Penzberg, Allemagne) a été utilisé. La préparation du mélange réactionnel était faite suivant les procédures recommandées par le fournisseur.

Dans un volume réactionnel de 20 µl, on mélangeait 5·10³ copies du plasmide avec les amorces sens et reverse (0,5 µM), le « molecular beacon » (1 µM), 2 µl du vial 1 du kit (enzyme mix), 0.8 µl de MgCl₂ à 25 mM du kit et de l'eau PCR grade du kit. Le mélange réactionnel était ensuite introduit dans un tube capillaire et celui-ci était introduit dans le LightCycler. Pour chaque réaction d'amplification, un témoin était fait avec une sonde TaqMan (TM) à la place du « molecular beacon » selon l'invention (G8). Pour chaque réaction d'amplification, un témoin était fait avec ajout d'eau PCR grade du kit à la place du plasmide cible (contrôle négatif, "c-").

La réaction PCR consistait en une dénaturation initiale de 8 minutes à 95°C, suivie de 40 cycles à 95°C pendant 30 secondes, 40°C pendant 5 secondes et 60°C pendant 60 secondes. La lecture de la fluorescence se faisait à 530 nm, en un point à la fin de chaque étape dans chaque cycle.

Les résultats de fluorescence étaient analysés de façon à séparer les résultats par température, les normaliser et en faire une représentation graphique. Les graphiques obtenus pour les lectures à 40°C et 60°C servaient à vérifier l'hybridation de la sonde avec les amplicons pendant la réaction d'amplification. A 95°C, par contre, il n'y a pas d'hybridation possible entre la sonde et les amplicons. Toute augmentation de signal à 95°C, par rapport au contrôle négatif, ne pouvait être due qu'au clivage préalable de la sonde. Le graphique obtenu pour la lecture à 95°C servait donc à prouver le clivage ou l'absence de clivage de sonde par l'activité 5'-nucléase de l'enzyme Taq Polymérase (ou TaqPol).

### 1.d : résultats et discussion :

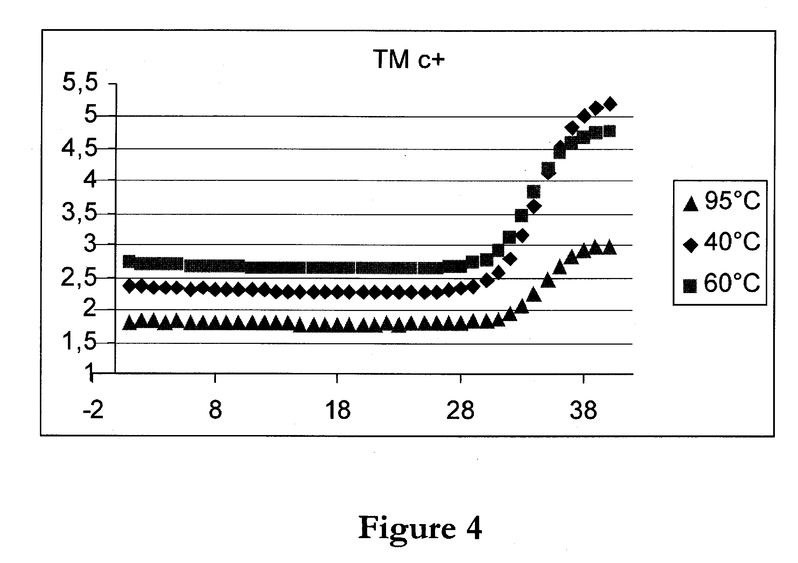

Les profils obtenus avec la sonde TaqMan TM en détection en temps réel est présenté dans les figures 3 et 4.

L'axe horizontal représente le nombre de cycles d'amplification, l'axe vertical représente la fluorescence détectée à 530 nm, à 40, 60°C et 95°C, dans chaque cycle. Les profils à 60 et 40°C démontrent que la sonde TaqMan TM détecte spécifiquement la présence des amplicons issus de la réaction enzymatique d'amplification *in vitro.* Les profils à 95°C démontrent que la sonde TM est clivée spécifiquement pendant la réaction d'amplification.

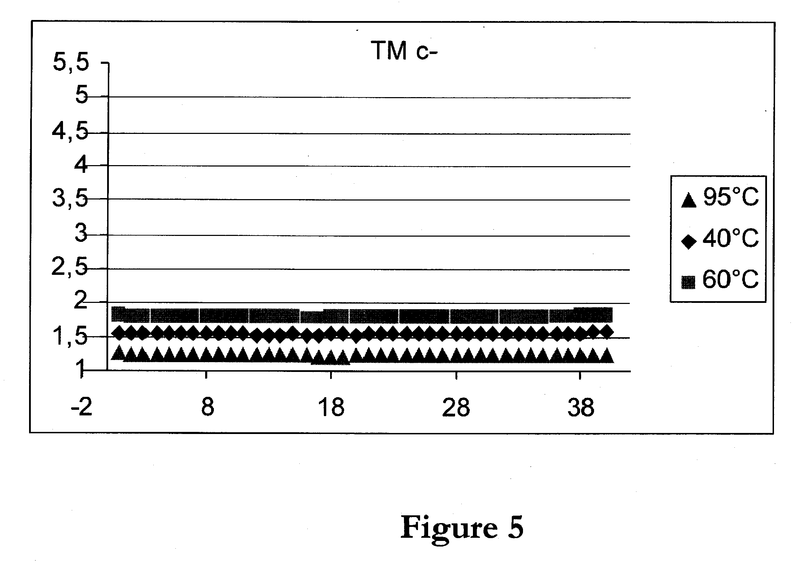

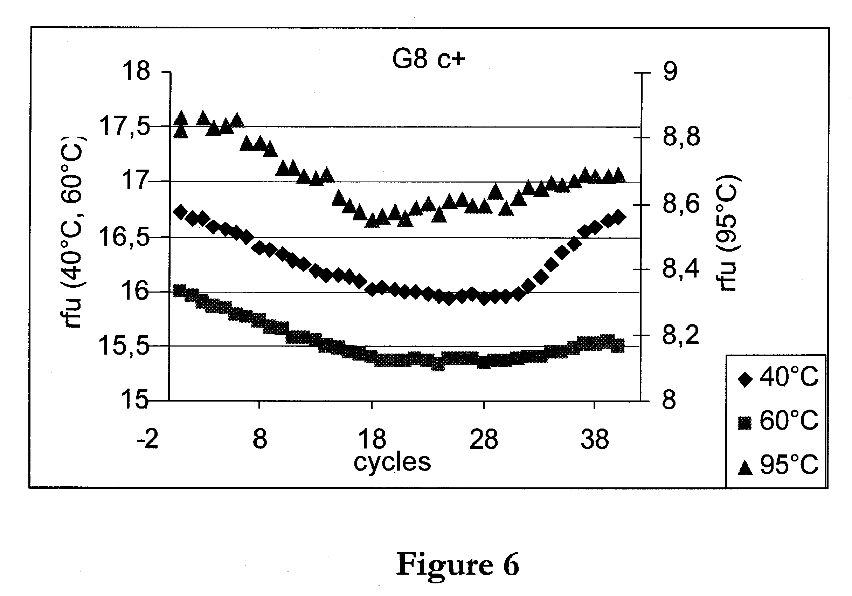

Les profils obtenus avec le « molecular beacon » G8 en détection en temps réel sont présentés dans les figures 5 et 6, dans laquelle l'axe horizontal représente le nombre de cycles d'amplification, l'axe vertical gauche représente la fluorescence détectée à 530 nm, à 40 et 60°C, dans chaque cycle ; l'axe vertical droit représente la fluorescence détectée à 530 nm, à 95°C, dans chaque cycle.

Les profils à 60 et 40°C démontrent que le « molecular beacon » G8 détectait spécifiquement la présence des amplicons issus de la réaction enzymatique d'amplification *in vitro.* Les profils à 95°C démontrent que le « molecular beacon » G8 n'était pas clivé dans les conditions dans lesquelles la sonde TaqMan TM est clivée.

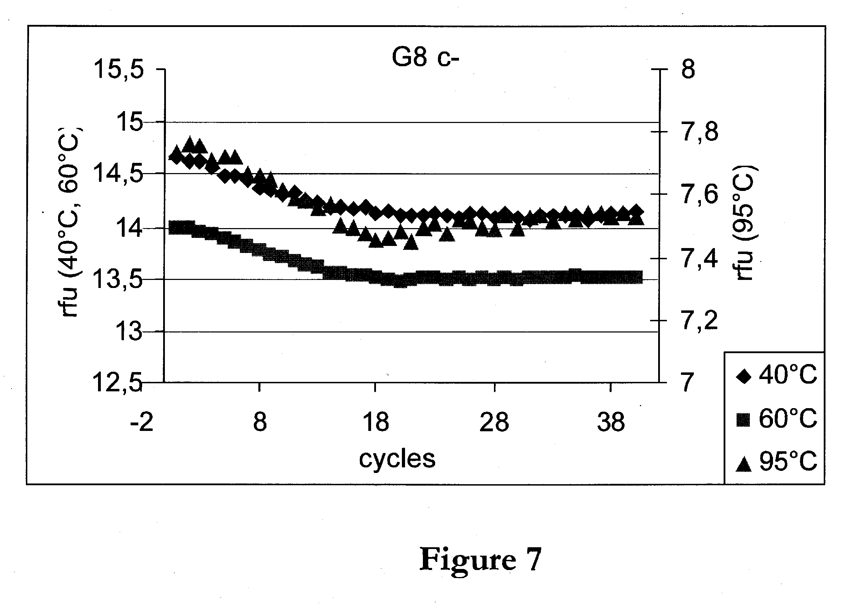

La figure 7 présente le comparatif des augmentations de fluorescence à 95°C pour la sonde TaqMan TM et pour le « molecular beacon » modifié G8. On pouvait observer une augmentation spécifique de la fluorescence seulement avec la sonde TM quand l'amplification a lieu. Quand l'amplification n'a pas lieu (contrôles négatifs) et quand le « molecular beacon » est modifié, G8, il n'y a pas de clivage par l'activité 5'-nuclease de la Taq Polymérase, et il n'y a donc pas d'augmentation de fluorescence.

Conclusion: Le « molecular beacon » selon l'invention G8 s'hybride spécifiquement avec les amplicons issus du cyclage sur lightcycler, donnant lieu à une détection en temps réel comparable à celle obtenue avec la sonde TaqMan TM. Le « molecular beacon » G8 n'est pas substrat pour le clivage par l'activité 5'-nuclease de la Taq Polymérase, au contraire que la sonde TaqMan TM.

### 2. Protection contre le clivage par l'activité 5'-nucléase par une séquence nucléotidique formant une structure en épingle à cheveux (« hairpin ») thermostable d'une sonde agissant par reconnaissance moléculaire moleculars beacon »)

L'objectif de cette expérience était de démontrer qu'un « molecular beacon » modifié dans son côté 5' par une séquence nucléotidique formant un « hairpin » thermostable n'était pas clivé par l'activité 5'-nucléase de la Taq Polymerase dans une réaction d'amplification enzymatique *in vitro.*

### 2.a : Définition de la cible, sondes et amorces

La cible est le plasmide pCITE avec insert de 1.2 Kb correspondant à une séquence génétique du virus hMPV (5·10³ copies par tube).
□ Amorce sens : 5'- CAT ATA AGC ATG CTA TAT TAA AAG AGT CTC -3'
□ Amorce reverse : 5'- CCT ATT TCT GCA GCA TAT TTG TAA TCA G -3'
□ « molecular beacon » (hp5): 5'-AT GCA TCG TTT TTC GAT GC AT(dT-FAM) CGA TGC AAC TGC AGT GAC ACC CTC ATC ATT GCA CAT CG (dT-Dabcyl)-3'
□ Sonde TaqMan (TM): 5'- FAM- TGC AAT GAT GAG GGT GTC ACT GCG GTT -TAMRA-3' - Cette séquence reconnaissait spécifiquement une séquence de l'amplicon généré dans l'amplification in vitro.

La partie soulignée de cette séquence reconnaissait spécifiquement une séquence de l'amplicon généré dans l'amplification in vitro. La partie de cette séquence comprise entre l'extrémité 5' et le fluorophore forme une structure en épingle à cheveux stable.

### 2.b : Synthèse de sondes et amorces

Les séquences de sondes et des amorces ont été synthétisées selon le protocole décrit dans l'exemple 1. b.

Le dT-Dabcyl est un nucléotide modifié qui porte une molécule Dabcyl attachée de façon covalente à la base T (ref. 10-1058, Glen Research, USA). Le dT-FAM est un nucléotide modifié qui porte une molécule Fluorescéine attachée de façon covalente à la base T (réf 10-1056, Glen Research, USA).

Le FAM est un fluorophore de type fluorescéine dont la fluorescence peut être détectée à 530 nm.

Le Dabcyl est une molécule aromatique qui empêche l'émission de fluorescence quand elle se trouve physiquement à proximité d'un fluorophore, c'est donc un extincteur, aussi appelé « quencher ».

La molécule TAMRA (dérivé de la tétraméthylrhodamine) est une molécule aromatique qui empêche l'émission de fluorescence de la fluorescéine par effet FRET lorsque ce deux molécules sont situées à proximité, par exemple sur une même séquence nucléotidique. L'effet FRET est proportionnel à la sixième puissance de la distance entre les deux molécules.

### 2.c. Amplification PCR avec détection en temps réel sur LightCycler:

Dans cet exemple, un kit d'amplification « LightCycler FastStart DNA Master Hybridization Probes » (Roche, Penzberg, Allemagne) a été utilisé. La préparation du mélange réactionnel était faite suivant les procédures recommandées par le fournisseur. Dans un volume réactionnel de 20 µl, on mélangeait 5.10³ copies du plasmide avec les amorces sens et reverse (0.5 µM), le « molecular beacon » modifié hp5 (1 µM), 2 µl du vial 1 du kit (enzyme mix), 0.8 µl de MgCl₂ à 25 mM du kit et de l'eau PCR grade du kit.

Le mélange réactionnel était ensuite introduit dans un tube capillaire et celui ci était introduit dans le LightCycler.

Pour chaque réaction d'amplification, un témoin était fait avec la sonde TaqMan (TM) à la place du « molecular beacon » modifié hp5.

Pour chaque réaction d'amplification, un témoin était fait avec ajout d'eau PCR grade du kit à la place du plasmide cible (contrôle négatif, "c-").

La réaction PCR consistait en une dénaturation initiale de 8 minutes à 95°C, suivie de 40 cycles à 95°C pendant 30 secondes, 40°C pendant 5 secondes et 60°C pendant 60 secondes.

La lecture de la fluorescence se faisait à 530 nm, en un point à la fin de chaque étape dans chaque cycle. Les résultats de fluorescence étaient ensuite analysés de façon à séparer les résultats par température, les normaliser et en faire une représentation graphique. Les graphiques obtenus pour les lectures à 40°C et 60°C servaient à vérifier l'hybridation de la sonde avec les amplicons pendant la réaction d'amplification. A 95°C, par contre, il n'y avait pas d'hybridation possible entre la sonde et les amplicons. Toute augmentation de signal à 95°C, par rapport au contrôle négatif, ne pouvait être due qu'au clivage de la sonde. Le graphique obtenu pour la lecture à 95°C sert donc à prouver le clivage ou non de la sonde par l'activité 5'-nucléase de la TaqPol.

### 2.d. Résultats et discussion

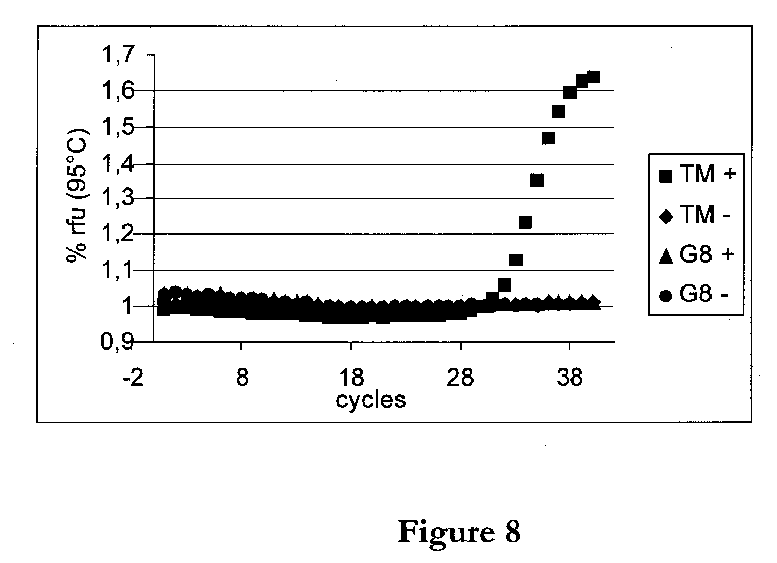

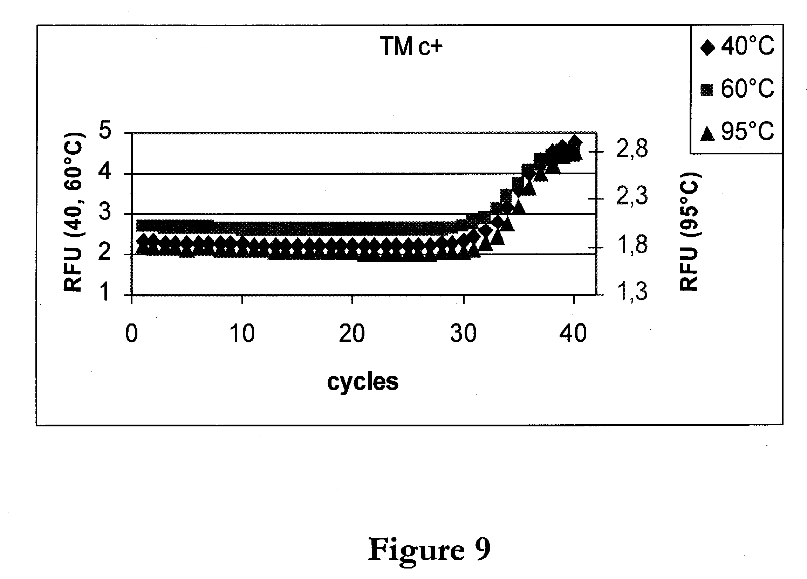

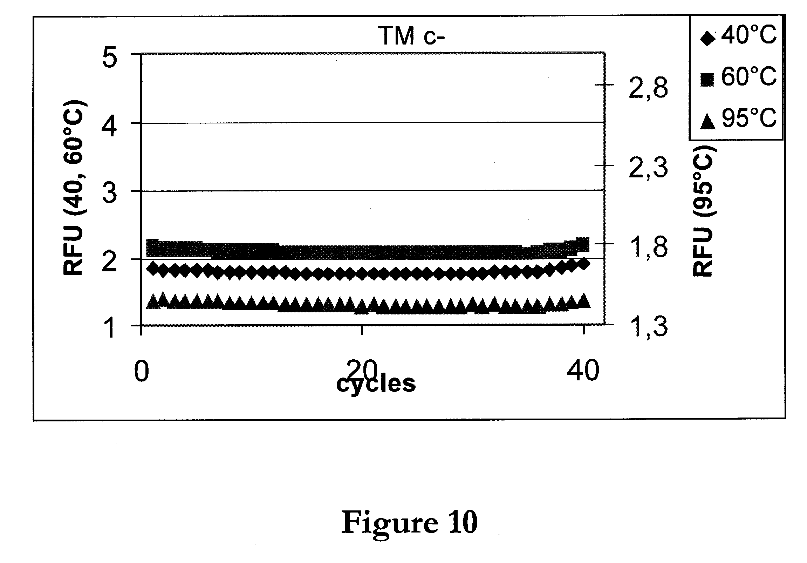

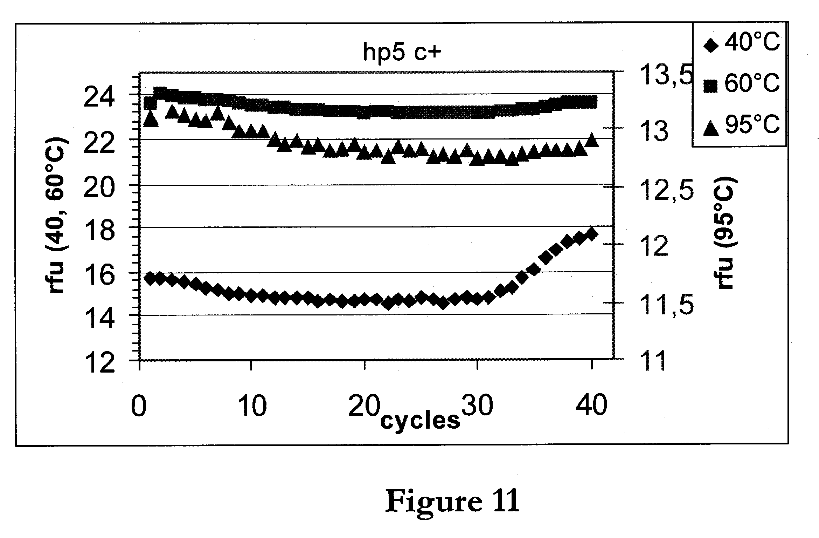

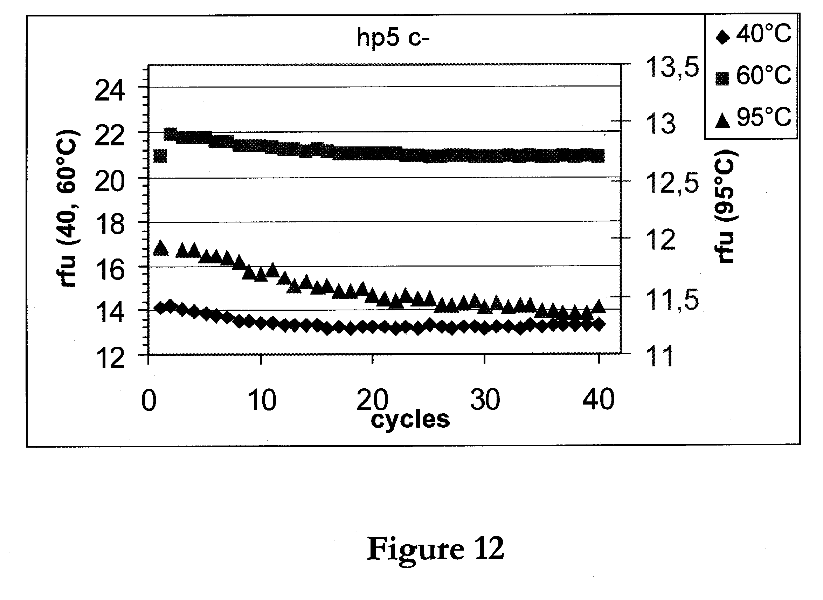

Les profils obtenus en détection en temps réel sont montrés dans les figures 8 à 12, dans lesquelles l'axe horizontal représente le nombre de cycles d'amplification, l'axe vertical gauche (fig. 8 à 11) représente la fluorescence détectée à 530 nm, à 40 et 60°C, dans chaque cycle, l'axe vertical droit (gauche dans la fig. 12) représente la fluorescence détectée à 530 nm, à 95°C, dans chaque cycle.

Les profils à 60 et 40°C démontrent que le « molecular beacon » selon l'invention hp5, ainsi que la sonde TM, détectaient spécifiquement la présence des amplicons issus de la réaction enzymatique d'amplification *in vitro.*

Les profils à 95°C démontraient que la sonde TM était clivée spécifiquement pendant la réaction d'amplification, alors que le hp5 n'était pas clivé dans les mêmes conditions.

La figure 12 présente le comparatif des augmentations de fluorescence à 95°C pour le « molecular beacon » modifié hp5, et pour la sonde TM.

On pouvait observer une augmentation spécifique de la fluorescence seulement lors de l'utilisation de la sonde TM quand l'amplification a lieu. Quand l'amplification n'a pas lieu (contrôles négatifs) et quand on utilise le « molecular beacon » modifié, il n'y avait pas de clivage par l'activité 5'-nuclease de la Taq Polymérase, et il n'y avait donc pas d'augmentation de fluorescence.

Conclusion: Le « molecular beacon » modifié hp5 s'hybridait spécifiquement avec les amplicons issus du cyclage sur lightcycler, donnant lieu à une détection en temps réel comparable à celle obtenue avec la sonde TaqMan TM. Le « molecular beacon » modifié hp5 n'était pas clivé par l'activité 5'-nucléase de la Taq Polymérase, contrairement à la sonde TaqMan.

### 3. Protection contre le clivage par l'activité 5'-nucléase par une séquence nucléotidique formant une structure en épingle à cheveux (« hairpin ») thermostable d'une sonde agissant par reconnaissance moléculaire (« molecular beacon »)

L'objectif de cette expérience était de démontrer qu'un « molecular beacon » modifié dans son côté 5' par une séquence nucléotidique formant un hairpin thermostable n'était pas clivé par l'activité 5'-nucléase de la Taq Polymérase dans une réaction d'amplification enzymatique *in vitro.*

Par rapport à l'exemple précèdent, le « molecular beacon » possède une molécule de Psoralène à son extrémité 5'. Cette molécule sert à rendre l' « hairpin » plus thermostable par rapport au « molecular beacon » sans le psoralène. Cette molécule s'intercale dans la structure en épingle à cheveux à laquelle elle est rattachée, et stabilise cette structure lui permettant de rester stable à la température de polymérisation.

### 3.a : Définition de la cible, sondes et amorces

La cible est le plasmide pCITE avec insert de 1,2 Kb correspondant à une séquence génétique du virus hMPV. 5·10³ copies par tube.

Les séquences utilisées dans cet exemple étaient les suivantes :
○ Amorce sens : 5'- CAT ATA AGC ATG CTA TAT TAA AAG AGT CTC - 3'
○ Amorce reverse : 5'- CCT ATT TCT GCA GCA TAT TTG TAA TCA G -3'
○ «"molecular beacon"» (hp2): 5'-Pso-GCA TCG TTT TTC GAT GC(dT-FAM)-T CGA TGC AAC TGC AGT GAC ACC CTC ATC ATT GCA CAT CG (dT-Dabcyl)-3'
○ « molecular beacon » contrôle (MBnm): 5'- (dT-FAIVI)-CGA TGC AAC TGC AGT GAC ACC CTC ATC ATT GCA CAT CG (dT-Dabcyl)-3' - Cette séquence reconnaissait spécifiquement une séquence de l'amplicon généré dans l'amplification in vitro. La partie soulignée de cette séquence reconnaissait spécifiquement une séquence de l'amplicon généré dans l'amplification in vitro.

### 3.b : Synthèse de sondes et amorces

Les séquences de sondes et des amorces ont été synthétisées selon le protocole décrit dans l'exemple 1. b.

"Pso" est une molécule de Psoralène qui est intégrée dans la séquence pendant la synthèse automatique à partir du phosphoramidite commercial (réf. 10-1982, Glen Research, USA. Cette molécule s'intercale dans la structure en épingle à cheveux à laquelle elle est rattachée, et stabilise cette structure lui permettant de rester stable à la température de polymérisation.

Le dT-FAM est un nucléotide modifié qui porte une molécule Fluorescéine attachée de façon covalente à la base T (réf 10-1056, Glen Research, USA).

Le dT-Dabcyl est un nucléotide modifié qui porte une molécule Dabcyl attachée de façon covalente à la base T (réf. 10-1058, Glen Research, USA).

Le FAM est un fluorophore de type fluorescéine dont la fluorescence peut être détectée à 530 nm.

Le Dabcyl est une molécule aromatique qui empêche l'émission de fluorescence quand elle se trouve physiquement à proximité du fluorophore FAM

### 3.c. Amplification PCR avec détection en temps réel sur Lightcycler:

Dans cet exemple, un kit d'amplification "LightCycler FastStart DNA Master Hybridization Probes" (Roche, Penzberg, Allemagne) a été utilisé. La préparation du mélange réactionnel était faite suivant les procédures recommandées par le fournisseur. Dans un volume réactionnel de 20 µl, on mélangeait 5.10³ copies du plasmide avec les amorces sens et reverse (0.5 µM), le « molecular beacon » selon l'invention hp2 (1 µM), 2 µl du vial 1 du kit (enzyme mix), 0.8 µl de MgC1₂ à 25 mM du kit et de l'eau PCR grade du kit.

Le mélange réactionnel était ensuite introduit dans un tube capillaire et celui ci était introduit dans le LightCycler.

Pour chaque réaction d'amplification, un témoin était fait avec le « molecular beacon » contrôle (MBnm) à la place du « molecular beacon » selon l'invention hp2.

Pour chaque réaction d'amplification, un témoin était fait avec ajout d'eau PCR grade du kit à la place du plasmide cible (contrôle negatif, "c-").

La réaction PCR consistait en une dénaturation initiale de 8 minutes à 95°C, suivie de 40 cycles à 95°C pendant 30 secondes, 40°C pendant 5 secondes et 60°C pendant 60 secondes.

La lecture de la fluorescence se faisait à 530 nm, en un point à la fin de chaque étape dans chaque cycle. Les résultats de fluorescence étaient ensuite analysés de façon à séparer les résultats par température, les normaliser et en faire une représentation graphique. Les graphiques obtenus pour les lectures à 40°C et 60°C servaient à vérifier l'hybridation de la sonde avec les amplicons pendant la réaction d'amplification. A 95°C, par contre, il n'y avait pas d'hybridation possible entre la sonde et les amplicons. Toute augmentation de signal à 95°C, par rapport au contrôle négatif, ne pouvait être due qu'au clivage de la sonde. Le graphique obtenu pour la lecture à 95°C sert donc à prouver le clivage ou non de la sonde par l'activité 5'-nucléase de la TaqPol.

### 3.d. Résultats et discussion

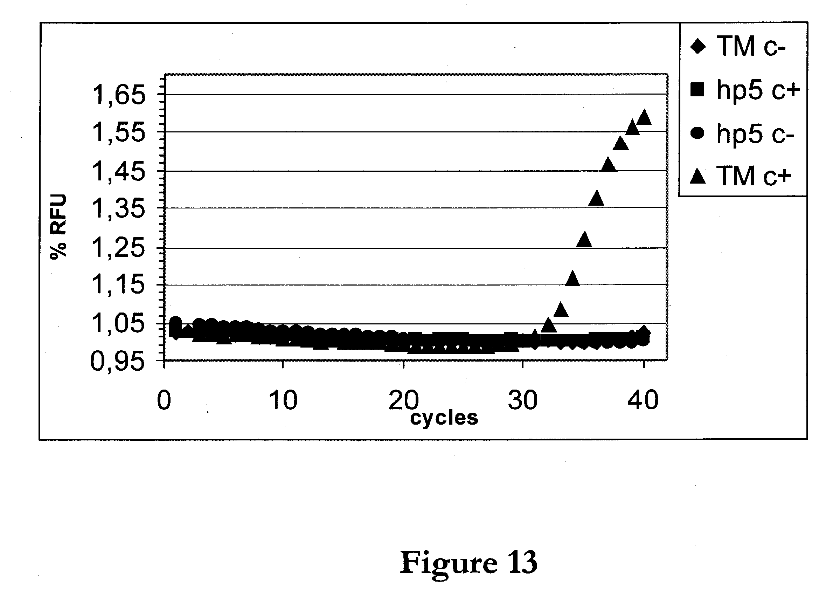

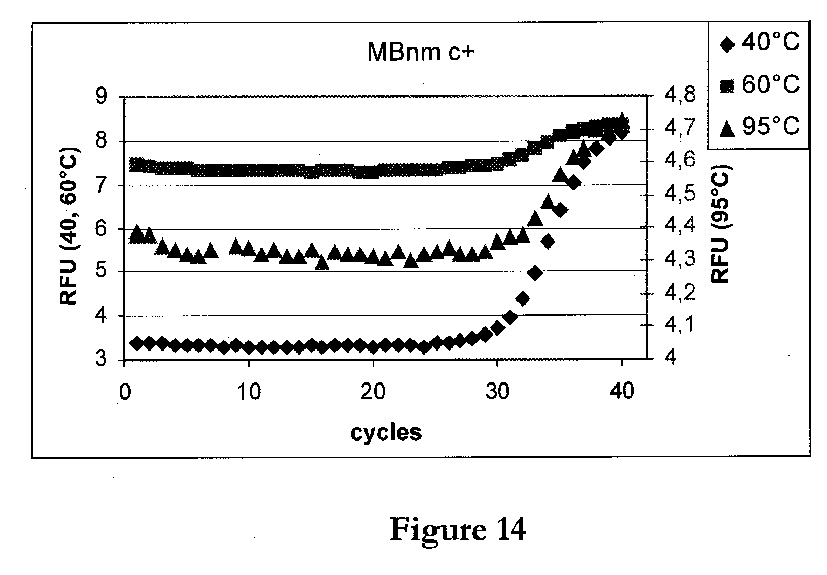

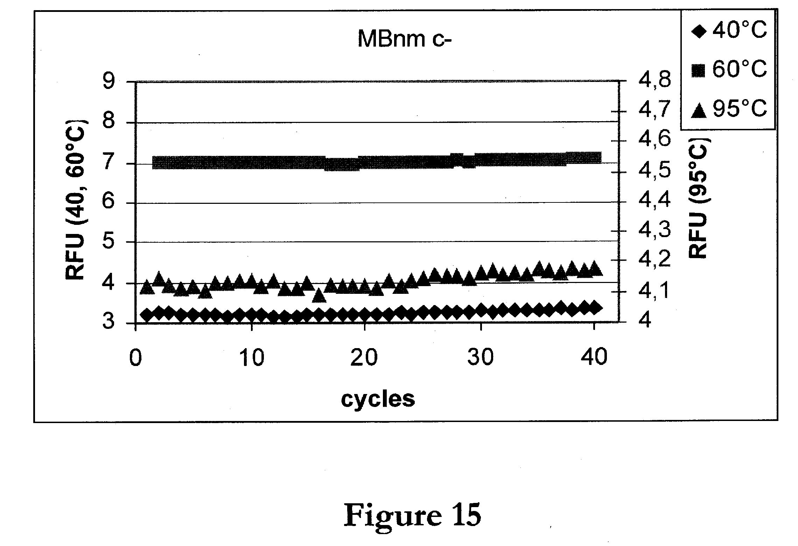

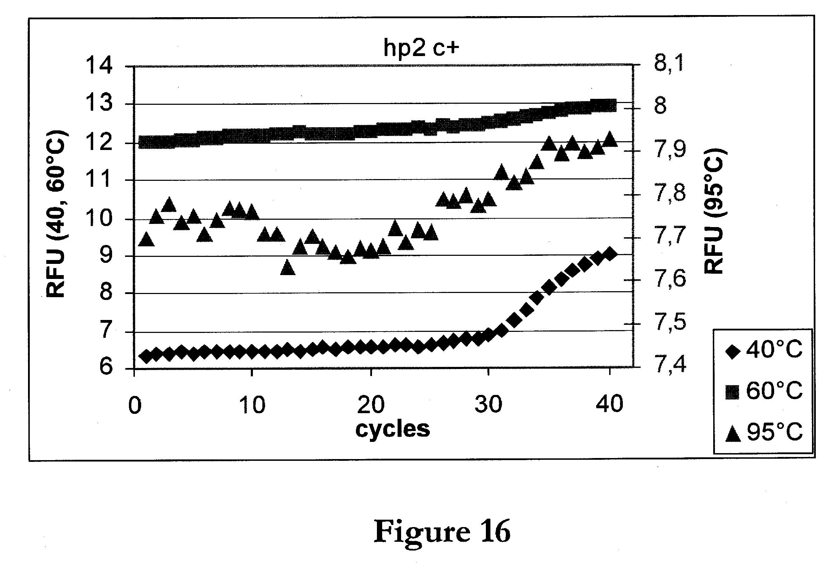

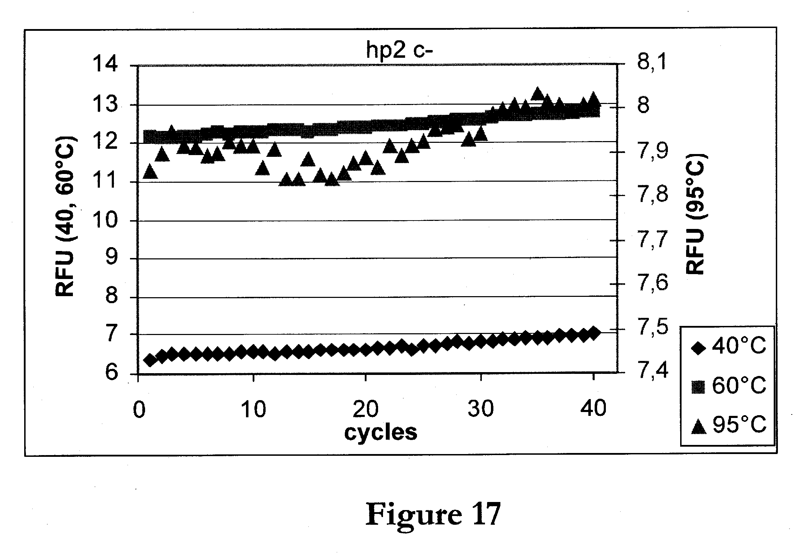

Les profils obtenus en détection en temps réel sont montrés dans les figures 13 à 17, dans lesquelles l'axe horizontal représente le nombre de cycles d'amplification, l'axe vertical gauche (fig 13 à 16) représente la fluorescence détectée à 530 nm, à 40 et 60°C, dans chaque cycle, l'axe vertical droit (gauche dans la fig. 17) représente la fluorescence détectée à 530 nm, à 95°C, dans chaque cycle.

Les profils à 60 et 40°C démontrent que le « molecular beacon » selon l'invention hp2 et le contrôle MBnm détectaient spécifiquement la présence des amplicons issus de la réaction enzymatique d'amplification *in vitro.*

Les profils à 95°C démontraient que le MBnm était clivé spécifiquement pendant la réaction d'amplification, alors que le hp2 n'était pas clivé dans les mêmes conditions.

La figure 17 présente le comparatif des augmentations de fluorescence à 95°C pour le « molecular beacon » non modifié MBnm et pour le modifié hp2.

On pouvait observer une augmentation spécifique de la fluorescence seulement lors de l'utilisation du « molecular beacon » MBnm quand l'amplification a lieu. Quand l'amplification n'a pas lieu (contrôles négatifs) et quand le « molecular beacon » est modifié selon l'invention, il n'y avait pas de clivage par l'activité 5'-nuclease de la Taq Polymerase, et il n'y avait donc pas d'augmentation de fluorescence. Conclusion: Le « molecular beacon » modifié hp2 s'hybridait spécifiquement avec les amplicons issus du cyclage sur lightcycler, donnant lieu à une détection en temps réel comparable à celle obtenue avec le « molecular beacon » non modifié MBnm. Le « molecular beacon » modifié hp2 n'était pas clivé par l'activité 5'-nuclease de la Taq Polymerase, contrairement au « molecular beacon » non modifié MBnm.

### 4. Exemple de protection contre le clivage par l'activité 5'-nuclease par une sequence nucléotidique formant une structure en épingle à cheveux (« hairpin ») thermostable d'une sonde agissant par reconnaissance moléculaire (« molecular beacon »).

L'objectif de cette expérience est de démontrer qu'un « molecular beacon » modifié dans son côté 5' par une séquence nucléotidique formant un « hairpin » thermostable n'est pas substrat pour le clivage par l'activité 5'-nucléase de la Taq Polymerase dans une réaction d'amplification enzymatique *in vitro.*

Par rapport aux exemples 2 et 3, ce « molecular beacon » modifié comporte un fluorophore dans son extrémité 5', au sein de la structure en épingle à cheveux, alors que dans les cas précédents la fluorescéine était localisé dans le premier segment du « molecular beacon » (voir fig. 2, droite).

### 4.a : Définition de la cible, sondes et amorces

La cible est le plasmide pCITE avec insert de 1.2 Kb correspondant à une séquence génétique du virus hMPV (5·10³ copies par tube).
○ Amorce sens : 5'- CAT ATA AGC ATG CTA TAT TAA AAG AGT CTC -3'
○ Amorce reverse : 5'- CCT ATT TCT GCA GCA TAT TTG TAA TCA G -3'
○ « molecular beacon » (hp4): 5'- FAM-GCA TCG TTT TTC GAT GC CGA TGC AAC TGC AGT GAC ACC CTC ATC ATT GCA CAT CG (dT-Dabcyl)-3'
○ Sonde TaqMan (TM): 5'- FAM- TGC AAT GAT GAG GGT GTC ACT GCG GTT -TAMRA-3' - Cette séquence reconnaissait spécifiquement une séquence de l'amplicon généré dans l'amplification in vitro.

La partie soulignée de cette séquence reconnaissait spécifiquement une séquence de l'amplicon généré dans l'amplification in vitro.

### 4.b : Synthèse de sondes et amorces

Les séquences de sondes et des amorces ont été synthétisées selon le protocole décrit dans l'exemple 1. b.

Le dT-Dabcyl est un nucléotide modifié qui porte une molécule Dabcyl attachée de façon covalente à la base T (ref. 10-1058, Glen Research, USA).

Le dT-FAM est un nucléotide modifié qui porte une molécule Fluorescéine attachée de façon covalente à la base T (ref 10-1056, Glen Research, USA).

Le FAM est un fluorophore de type fluorescéine dont la fluorescence peut être détectée à 530 nm.

Le Dabcyl est une molécule aromatique qui empêche l'émission de fluorescence quand elle se trouve physiquement à proximité d'un fluorophore, c'est donc un "extincteur", aussi appelé ""quencher"".

La molécule TAMRA (dérivé de la tetramethylrhodamine) est une molécule aromatique qui empêche l'émission de fluorescence de la fluorescéine par effet FRET lorsque ce deux molécules sont situées à proximité, par exemple sur une même séquence nucléotidique. L'effet FRET est proportionnel à la sixième puissance de la distance entre les deux molécules.

### 4.c. Amplification PCR avec détection en temps réel sur LightCycler:

Dans cet exemple, un kit d'amplification "LightCycler FastStart DNA Master Hybridization Probes" (Roche, Penzberg, Allemagne) a été utilisé. La préparation du mélange réactionnel était faite suivant les procédures recommandées par le fournisseur. Dans un volume réactionnel de 20 µl, on mélangeait 5.10³ copies du plasmide avec les amorces sens et reverse (0.5 µM), le « molecular beacon » hp4 (1 µM), 2 µl du vial 1 du kit (enzyme mix), 0.8 µl de MgCl₂ à 25 mM du kit et de l'eau PCR grade du kit.

Le mélange réactionnel était ensuite introduit dans un tube capillaire et celui-ci était introduit dans le LightCycler.

Pour chaque réaction d'amplification, un témoin était fait avec la sonde TaqMan (TM) à la place du « molecular beacon » modifié hp4.

Pour chaque réaction d'amplification, un témoin était fait avec ajout d'eau PCR grade du kit à la place du plasmide cible (contrôle négatif, "c-").

La réaction PCR consistait en une dénaturation initiale de 8 minutes à 95°C, suivie de 40 cycles à 95°C pendant 30 secondes, 40°C pendant 5 secondes et 60°C pendant 60 secondes.

La lecture de la fluorescence se faisait à 530 nm, en un point à la fin de chaque étape dans chaque cycle. Les résultats de fluorescence étaient ensuite analysés de façon à séparer les résultats par température, les normaliser et en faire une représentation graphique. Les graphiques obtenus pour les lectures à 40°C et 60°C servaient à vérifier l'hybridation de la sonde avec les amplicons pendant la réaction d'amplification. A 95°C, par contre, il n'y avait pas d'hybridation possible entre la sonde et les amplicons. Toute augmentation de signal à 95°C, par rapport au contrôle négatif, ne pouvait être due qu'au clivage de la sonde. Le graphique obtenu pour la lecture à 95°C sert donc à prouver le clivage ou non de la sonde par l'activité 5'-nucléase de la TaqPol.

### 4.d. Résultats et discussion

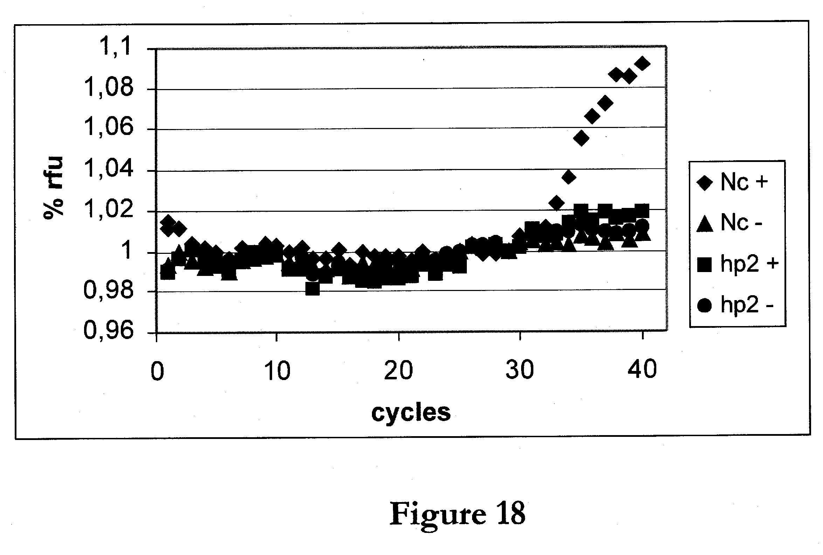

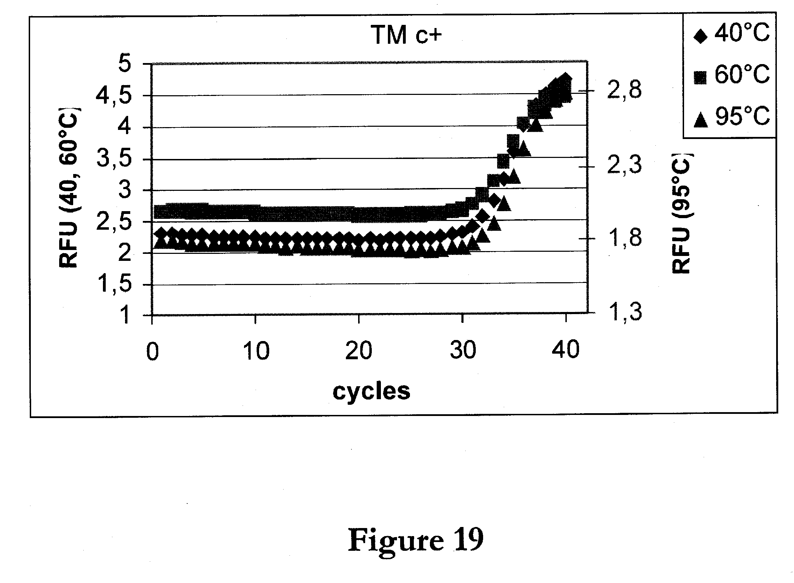

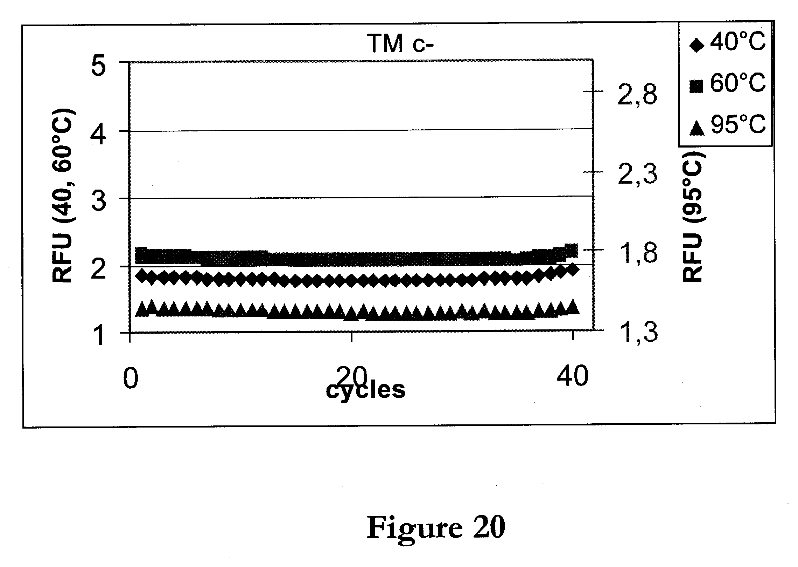

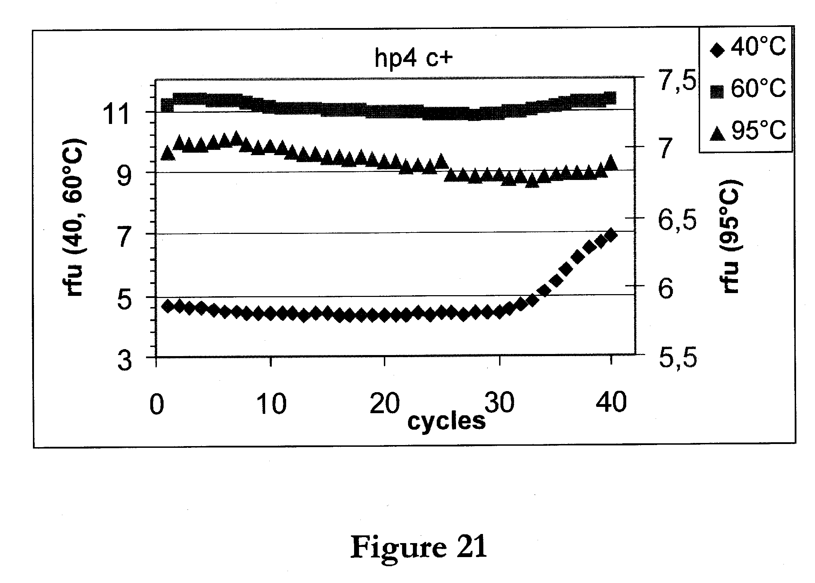

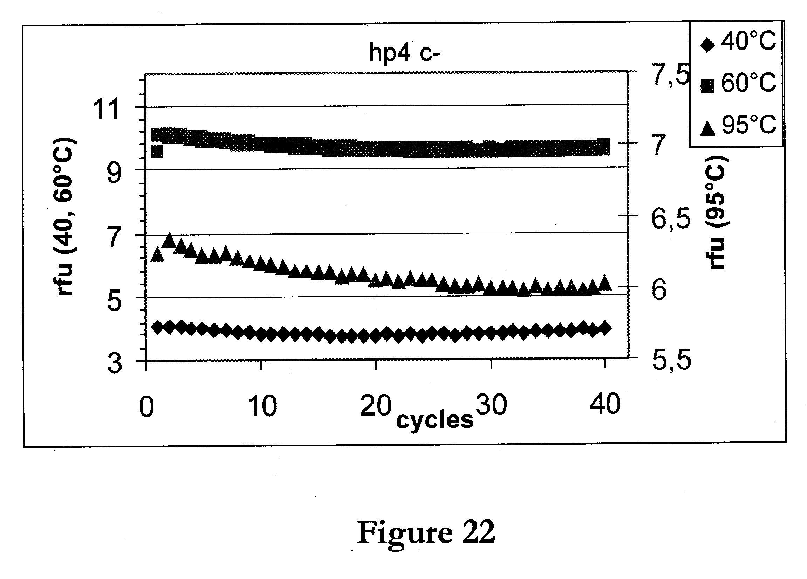

Les profils obtenus en détection en temps réel sont montrés dans les figures 18 à 22, dans lesquelles l'axe horizontal représente le nombre de cycles d'amplification, l'axe vertical gauche (fig. 18 à 21) représente la fluorescence détectée à 530 nm, à 40 et 60°C, dans chaque cycle, l'axe vertical droit (gauche dans la fig. 22) représente la fluorescence détectée à 530 nm, à 95°C, dans chaque cycle.

Les profils à 60 et 40°C démontrent que le « molecular beacon » modifié hp4, ainsi que la sonde TM, détectait spécifiquement la présence des amplicons issus de la réaction enzymatique d'amplification *ïn vitro.*

Les profils à 95°C démontraient que la sonde TM était clivée spécifiquement pendant la réaction d'amplification, alors que le hp4 n'était pas clivé dans les mêmes conditions.

La figure 22 présente le comparatif des augmentations de fluorescence à 95°C pour le « molecular beacon » modifié hp4 et pour la sonde TM.

On pouvait observer une augmentation spécifique de la fluorescence seulement lors de l'utilisation de la sonde TM quand l'amplification a lieu. Quand l'amplification n'a pas lieu (contrôles négatifs) et quand on utilise le « molecular beacon » modifié, il n'y avait pas de clivage par l'activité 5'-nuclease de la Taq Polymerase, et il n'y avait donc pas d'augmentation de fluorescence.

Conclusion: Le « molecular beacon » selon l'invention hp4 s'hybridait spécifiquement avec les amplicons issus du cyclage sur lightcycler, donnant lieu à une détection en temps réel comparable à celle obtenue avec la sonde TaqMan TM. Le « molecular beacon » modifié selon l'invention hp4 n'était pas clivé par l'activité 5'-nuclease de la Taq Polymerase, contrairement à la sonde TaqMan.

### 5. Exemple de protection contre le clivage par l'activité 5'-nuclease par une séquence nucléotidique formant une structure en épingle à cheveux (« hairpin ») thermostable d'une sonde agissant par reconnaissance moléculaire (« molecular beacon »).

L'objectif de cette expérience est de démontrer qu'un « molecular beacon » modifié dans son côté 5' par une séquence nucléotidique formant un hairpin thermostable n'est pas substrat pour le clivage par l'activité 5'-nucléase de la Taq Polymerase dans une réaction d'amplification enzymatique *in vitro.*

Par rapport aux exemples 2 et 3, ce « molecular beacon » modifié comporte un fluorophore dans son extrémité 5', au sein de la structure en épingle à cheveux, alors que dans les exemples 2 et 3 la fluorescéine était localisé dans le premier fragment du « molecular beacon » (voir fig. 2, droite).

Par rapport à l'exemple 4, ce « molecular beacon » modifié comporte un nucléotide T entre le premier fragment du « molecular beacon » et la structure en épingle à cheveux. Ce nucléotide éloigne la double hélice formée par la structure en épingle à cheveux et celle formée par les premier et troisième fragments du « molecular beacon », ce qui diminue les interactions réciproques.

### 5.a : Définition de la cible, sondes et amorces

La cible est le plasmide pCITE avec insert de 1.2 Kb correspondant à une séquence génétique du virus hMPV. 5·10³ copies par tube.
○ Amorce sens : 5'- CAT ATA AGC ATG CTA TAT TAA AAG AGT CTC -3'
○ Amorce reverse : 5'- CCT ATT TCT GCA GCA TAT TTG TAA TCA G -3'
○ « molecular beacon » (hp3): 5'- FAM-GCA TCG TTT TTC GAT GCT CGA TGC AAC TGC AGIT GAC ACC CTC ATC ATT GCA CAT CG (dT-Dabcyl)-3'
○ Sonde TaqMan (TM) : 5'- FAM- TGC AAT GAT GAG GGT GTC ACT GCG GTT -TAMRA-3' - Cette séquence reconnaissait spécifiquement une séquence de l'amplicon généré dans l'amplification in vitro.

La partie soulignée de cette séquence reconnaissait spécifiquement une séquence de l'amplicon généré dans l'amplification in vitro.

### 5.b : Synthèse de sondes et amorces

Les séquences de sondes et des amorces ont été synthétisées selon le protocole décrit dans l'exemple 1. b.

Le dT-Dabcyl est un nucléotide modifié qui porte une molécule Dabcyl attachée de façon covalente à la base T (réf. 10-1058, Glen Research, USA).

Le dT-FAM est un nucléotide modifié qui porte une molécule Fluorescéine attachée de façon covalente à la base T (réf 10-1056, Glen Research, USA).

Le FAM est un fluorophore de type fluorescéine dont la fluorescence peut être détectée à 530 nm.

Le Dabcyl est une molécule aromatique qui empêche l'émission de fluorescence quand elle se trouve physiquement à proximité d'un fluorophore, c'est donc un extincteur, aussi appelé « quencher ».

La molécule TAMRA (dérivé de la tétraméthylrhodamine) est une molécule aromatique qui empêche l'émission de fluorescence de la fluorescéine par effet FRET lorsque ce deux molécules sont situées à proximité, par exemple sur une même séquence nucléotidique. L'effet FRET est proportionnel à la sixième puissance de la distance entre les deux molécules.

### 5.c. Amplification PCR avec détection en temps réel sur LightCycler:

Dans cet exemple, un kit d'amplification « LightCycler FastStart DNA Master Hybridization Probes » (Roche, Penzberg, Allemagne) a été utilisé. La préparation du mélange réactionnel était faite suivant les procédures recommandées par le fournisseur. Dans un volume réactionnel de 20 µl, on mélangeait 5.10³ copies du plasmide avec les amorces sens et reverse (0.5 µM), le « molecular beacon » hp3 (1 µM), 2 µl du vial 1 du kit (enzyme mix), 0.8 µl de MgCl₂ à 25 mM du kit et de l'eau PCR grade du kit.

Le mélange réactionnel était ensuite introduit dans un tube capillaire et celui-ci était introduit dans le LightCycler.

Pour chaque réaction d'amplification, un témoin était fait avec la sonde TaqMan (TM) à la place du « molecular beacon » selon l'invention hp3.

Pour chaque réaction d'amplification, un témoin était fait avec ajout d'eau PCR grade du kit à la place du plasmide cible (contrôle négatif, « c- »).

La réaction PCR consistait en une dénaturation initiale de 8 minutes à 95°C, suivie de 40 cycles à 95°C pendant 30 secondes, 40°C pendant 5 secondes et 60°C pendant 60 secondes.

La lecture de la fluorescence se faisait à 530 nm, en un point à la fin de chaque étape dans chaque cycle. Les résultats de fluorescence étaient ensuite analysés de façon à séparer les résultats par température, les normaliser et en faire une représentation graphique. Les graphiques obtenus pour les lectures à 40°C et 60°C servaient à vérifier l'hybridation de la sonde avec les amplicons pendant la réaction d'amplification. A 95°C, par contre, il n'y avait pas d'hybridation possible entre la sonde et les amplicons. Toute augmentation de signal à 95°C, par rapport au contrôle négatif, ne pouvait être due qu'au clivage de la sonde. Le graphique obtenu pour la lecture à 95°C sert donc à prouver le clivage ou non de la sonde par l'activité 5'-nucléase de la TaqPol.

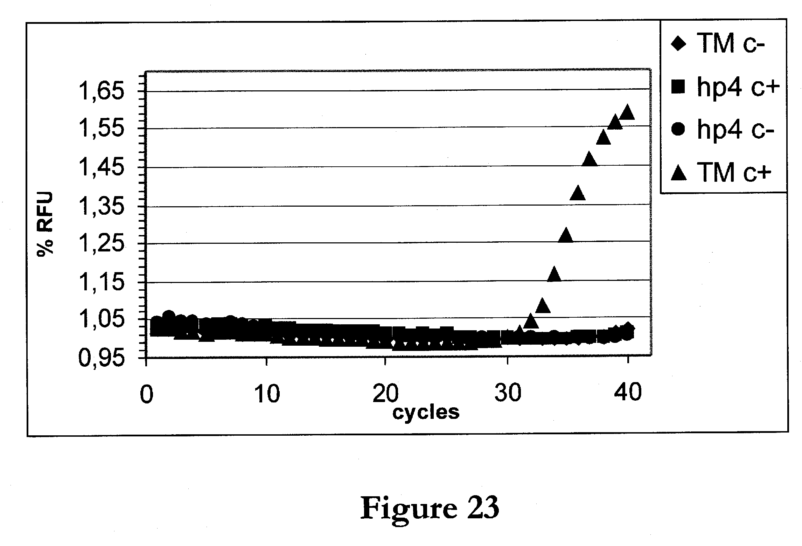

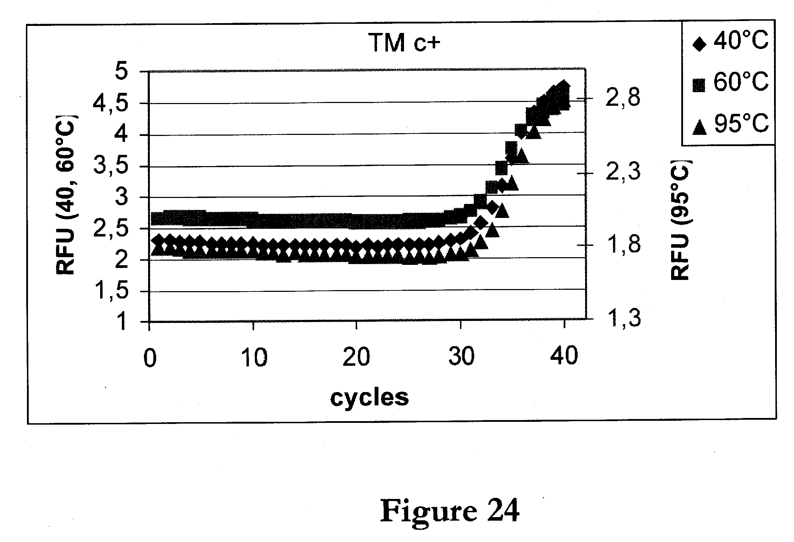

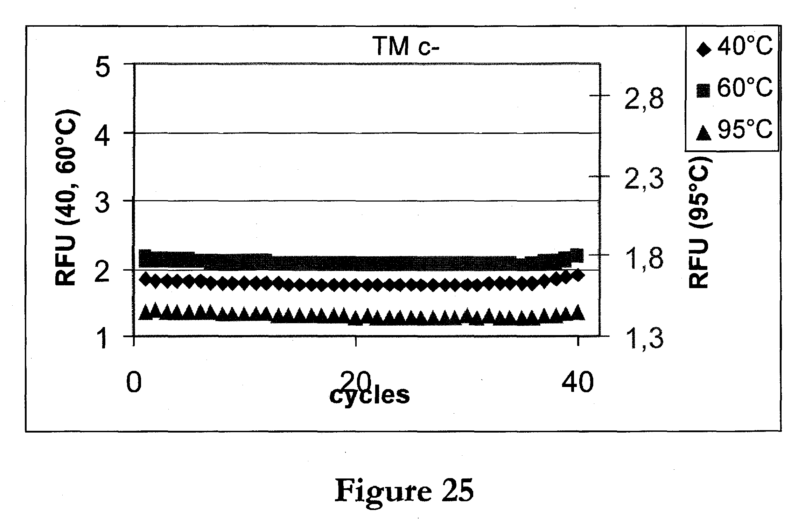

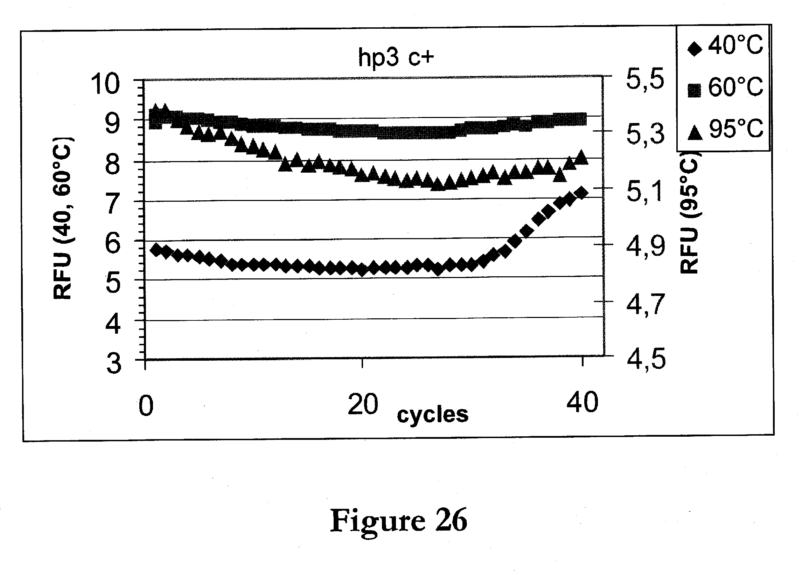

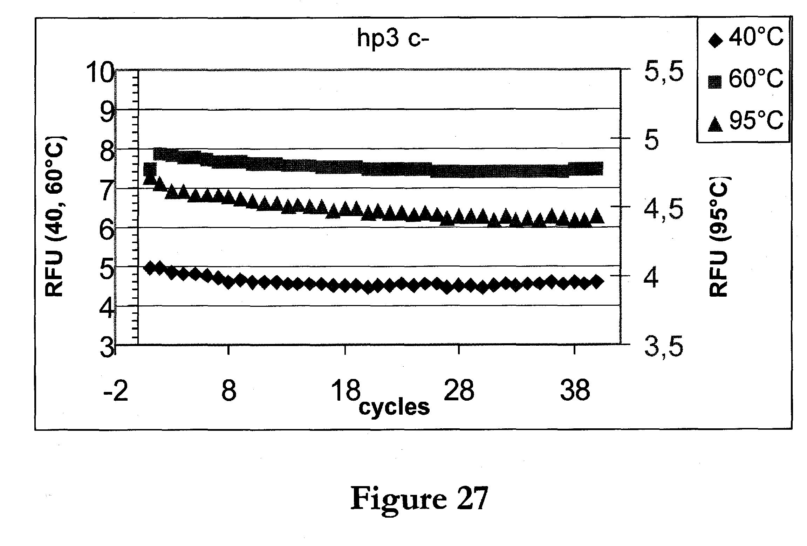

Les profils obtenus en détection en temps réel sont montrés dans les figures 23 à 27, dans lesquelles l'axe horizontal représente le nombre de cycles d'amplification, l'axe vertical gauche (fig. 23 à 26) représente la fluorescence détectée à 530 nm, à 40 et 60°C, dans chaque cycle, l'axe vertical droit (gauche dans la fig. 27) représente la fluorescence détectée à 530 nm, à 95°C, dans chaque cycle.

Les profils à 60 et 40°C démontrent que le « molecular beacon » modifié hp3, ainsi que la sonde TM, détectait spécifiquement la présence des amplicons issus de la réaction enzymatique d'amplification *in vitro.*

Les profils à 95°C démontraient que la sonde TM était clivée spécifiquement pendant la réaction d'amplification, alors que le hp3 n'était pas clivé dans les mêmes conditions.

La figure 27 présente le comparatif des augmentations de fluorescence à 95°C pour le « molecular beacon » modifié hp3 et pour la sonde TM.

On pouvait observer une augmentation spécifique de la fluorescence seulement lors de l'utilisation de la sonde TM quand l'amplification a lieu. Quand l'amplification n'a pas lieu (contrôles négatifs) et quand on utilise le « molecular beacon » modifié, il n'y avait pas de clivage par l'activité 5'-nuclease de la Taq Polymerase, et il n'y avait donc pas d'augmentation de fluorescence.

Conclusion: Le « molecular beacon» modifié hp3 s'hybridait spécifiquement avec les amplicons issus du cyclage sur lightcycler, donnant lieu à une détection en temps réel comparable à celle obtenue avec la sonde TaqMan TM. Le « molecular beacon» modifié hp3 n'était pas clivé par l'activité 5'-nuclease de la Taq Polymerase, contrairement à la sonde TaqMan.

## Revendications

1. Utilisation d'au moins une sonde de détection agissant par reconnaissance moléculaire d'une séquence cible, la sonde comprenant successivement dans le sens 5'-3'
■ un premier segment nucléotidique comprenant une structure stériquement encombrante à son extrémité 5', et
■ un deuxième segment nucléotidique, complémentaire de la séquence cible, pour bloquer une activité 5' nucléase d'une enzyme polymérase thermostable lors d'une Réaction de Polymérisation en Chaîne, la structure stériquement encombrante étant une structure en double brin.

2. Utilisation, selon la revendication 1, **caractérisée en ce que** la structure stériquement encombrante est une structure en épingle à cheveux.

3. Utilisation, selon l'une quelconque des revendications 1 ou 2, **caractérisée en ce que** la sonde comprend en outre, en aval dudit deuxième segment nucléotidique, un troisième segment nucléotidique, capable de s'hybrider sur ledit premier segment nucléotidique.

4. Utilisation, selon l'une quelconque des revendications 1 à 3, **caractérisée en ce que** la sonde est marquée, à une extrémité, par un fluorophore et à l'autre extrémité, par un extincteur.

5. Utilisation, selon la revendication 4, **caractérisée en ce que** ledit fluorophore est une fluorescéine.

6. Utilisation, selon l'une quelconque des revendications 1 à 5, **caractérisée en ce que** ledit premier segment comprend de 3 à 8 nucléotides.

7. Utilisation, selon l'une quelconque des revendications 1 à 6, **caractérisée en ce que** ledit deuxième segment comprend de 10 à 35 nucléotides.

8. Utilisation, selon la revendication 3, d'une part, et selon l'une quelconque des revendications 4 à 7, d'autre part, **caractérisée en ce que** ledit troisième segment comprend de 3 à 8 nucléotides.

9. Utilisation, selon l'une quelconque des revendications 1 à 8, **caractérisée en ce que** la structure stériquement encombrante a au moins la taille d'une double hélice d'ADN, c'est-à-dire un diamètre minimum d'au moins 20 angstrôms.

10. Procédé pour détecter un matériel nucléique dans un échantillon biologique comprenant les étapes suivantes :
a) on extrait le matériel nucléique d'un échantillon biologique,
b) on amplifie le matériel nucléique pour obtenir des amplicons d'au moins une séquence cible du matériel nucléique en présence d'une enzyme polymérase,
c) on utilise, simultanément à l'étape b) d'amplification ou postérieurement à l'étape b), au moins une sonde de détection agissant par reconnaissance moléculaire, comprenant successivement dans le sens 5'-3' :
■ un premier segment nucléotidique comprenant une structure stériquement encombrante à son extrémité 5', et
■ un deuxième segment nucléotidique, complémentaire de la séquence cible, pour bloquer une activité 5' nucléase d'une enzyme polymérase thermostable lors d'une Réaction de Polymérisation en Chaîne, la structure stériquement encombrante est une structure en double brin, et
d) on détecte la présence desdits amplicons.

11. Procédé, selon la revendication 10, **caractérisée en ce que** la détection des amplicons lors d'une Réaction de Polymérisation en Chaîne, s'effectue en temps réel.

12. Procédé, selon la revendication 10, **caractérisée en ce que** la détection des amplicons lors d'une Réaction de Polymérisation en Chaîne, s'effectue lors de mesures en « end-point » post-amplification.


SEQUENCE LISTING

<110>  bioMérieux SA

<120>  Nouvelle sonde de détection agissant par reconnaissance
       moléculaire

<130>  FR 06 51440

<160>  10

<170>  PatentIn version 3.3

<210>  1
<211>  30
<212>  DNA
<213>  HMPV

<400>  1
catataagca tgctatatta aaagagtctc                                        30


<210>  2
<211>  28
<212>  DNA
<213>  HMPV

<400>  2
cctatttctg cagcatattt gtaatcag                                          28


<210>  3
<211>  38
<212>  DNA
<213>  HMPV

<400>  3
cgatgcaact gcagtgacac cctcatcatt gcacatcg                               38


<210>  4
<211>  27
<212>  DNA
<213>  HMPV

<400>  4
tgcaatgatg agggtgtcac tgcggtt                                           27


<210>  5
<211>  30
<212>  DNA
<213>  HMPV

<400>  5
catataagca tgctatatta aaagagtctc                                        30


<210>  6
<211>  28
<212>  DNA
<213>  HMPV

<400>  6
cctatttctg cagcatattt gtaatcag                                          28


<210>  7
<211>  59
<212>  DNA
<213>  HMPV

<400>  7

atgcatcgtt tttcgatgca tcgatgcaac tgcagtgaca ccctcatcat tgcacatcg 59

<210> 8
<211> 27
<212> DNA
<213> HMPV

<400> 8
tgcaatgatg agggtgtcac tgcggtt 27

<210> 9
<211> 56
<212> DNA
<213> HMPV

<400> 9
gcatcgtttt tcgatgctcg atgcaactgc agtgacaccc tcatcattgc acatcg 56

<210> 10
<211> 38
<212> DNA
<213> HMPV

<400> 10
cgatgcaact gcagtgacac cctcatcatt gcacatcg 38